(19) **Europäisches Patentamt / European Patent Office / Office européen des brevets**

(11) **EP 4 443 191 B1**

(12) **EUROPEAN PATENT SPECIFICATION**

(45) Date of publication and mention
of the grant of the patent:
**10.09.2025 Bulletin 2025/37**

(21) Application number: **23166452.5**

(22) Date of filing: **04.04.2023**

(51) International Patent Classification (IPC):
*G01S 7/292* (2006.01)     *G01S 7/41* (2006.01)
*G01S 13/10* (2006.01)     *G01S 13/42* (2006.01)
*G01S 13/58* (2006.01)     *G01S 13/72* (2006.01)

(52) Cooperative Patent Classification (CPC):
**G01S 7/2923; G01S 7/415; G01S 7/417;**
**G01S 13/10; G01S 13/42; G01S 13/582;**
**G01S 13/726**

(54) **APPARATUS, SENSOR SYSTEM, ELECTRONIC DEVICE AND METHOD**

VORRICHTUNG, SENSORSYSTEM, ELEKTRONISCHE VORRICHTUNG UND VERFAHREN

APPAREIL, SYSTÈME DE CAPTEUR, DISPOSITIF ÉLECTRONIQUE ET PROCÉDÉ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**09.10.2024 Bulletin 2024/41**

(73) Proprietor: **Infineon Technologies AG**
**85579 Neubiberg (DE)**

(72) Inventor: **MELNIKOV, Alexandr**
**SAR PRC, Hong Kong (HK)**

(74) Representative: **2SPL Patentanwälte PartG mbB**
**Landaubogen 3**
**81373 München (DE)**

(56) References cited:
**WO-A2-2022/101390      CN-A- 109 581 343**
**CN-A- 115 015 867       US-A1- 2015 369 911**

## Description

### Field

Examples relate to signal processing of sensor data. In particular, examples relate to an apparatus, a sensor system, an electronic device and a computer-implemented method.

### Background

A sensor may generate a receive signal based on reflections of a transmitted signal.

WO 2022/101390 A2 proposes a method of optimizing a dataset from a phased-array radar to detect micro body movement of subjects due to heartbeat or respiration. The radar creates a 3D point-cloud data set, which is fed into an object identification algorithm to create at least one group of datapoints characterized by their proximity and their pattern of movement. The at least one group belonging to a single subject. Aggregating the datapoints of the group to form a velocity signal. A focus adjustment of said radar is done by dynamically focusing on a centroid of said at least one group.

US 2015/369911 A1 proposes a method and system for using radar to provide non-contact detection of human breathing at a distance. Radar data is processed to remove clutter and enable detection of a breathing signal. The breathing signal can be used to determine breathing rate or posture of the target. Static obstructions can be removed from the radar data to identify target breathing signal. The system and method can be applicable to emergency response, home or institutional monitoring applications and for detection of medical conditions.

CN 115 015 867 A proposes an identity recognition and fall detection method based on an ultra wide band radar. The method comprises the following steps: preprocessing a radar echo signal; according to the breathing cycle dynamic segmentation signal, acquiring breathing characteristics of the target; some features are extracted from the radar echo signal envelope to describe a target radar cross section (RCS), namely the posture features of the target; according to the time-varying distance information acquired by the radar, extracting a mean value and a variance of the distance, the speed and the acceleration as target motion features for identity recognition; and performing fall detection by using the approximate entropy. According to the method, by extracting the multi-modal features of the breathing feature, the posture feature and the motion feature of the target in the radar echo signal, the average accuracy of identity recognition by applying the random forest is 86.3%, and compared with other methods, the accuracy is higher. Through the fall event detection method based on the approximate entropy, the signal complexity change in the time continuous signal caused by the fall event is detected, the fall detection accuracy is 96.53%, and a new technology is provided for fall detection.

CN 109 581 343 A proposes a multi-radar networking device and multi-directional target detection method. The device comprises multiple radars and an upper computer; the radars are interactively connected with the upper computer; each radar is provided with a GPS module, an electronic compass module and an automatic learning module, the radar is used for detecting a target and reporting the target information of the radar to the upper computer, and the GPS module is used for positioning the longitude and latitude information of the radar and reporting the longitude and latitude information of the radar to the upper computer via the radar; the electronic compass module is used for detecting the azimuth information of the radar and reporting the azimuth information of the radar to the upper computer via the radar; and the automatic learning module is used for automatically learning a surrounding environment and setting different thresholds for different distance units of the radar. Detection in multiple directions is achieved, the problem that the azimuth range is limited when the radar is used for detecting the target is solved, and the dead angle of the radar is avoided.

Conventionally, it may be a complex task to discriminate a static person against a moving or vibrating object based on the receive signal, since the static person and a slowly moving object may exhibit a similar signal signature.

Hence, there may be a demand for improved signal processing of sensor data.

### Summary

The demand is satisfied by the subject matter of the independent claims. Further beneficial embodiments are given by the dependent claims.

Some aspects of the present disclosure relate to an apparatus, comprising processing circuitry configured to process data indicating a receive signal of a sensor through isolating a locally stationary signal component of the receive signal from at least one of a stochastic and a deterministic signal component of the receive signal. According to the invention, the locally stationary signal component indicates a breathing motion in a field of view of the sensor. The processing circuitry is configured to process the data through selecting at least two frames from a plurality of frames of the data based on a predefined breathing rate assumed for the breathing motion and feeding the selected frames into a moving target indicator.

Some aspects of the present disclosure relate to a sensor system, comprising an apparatus as described herein, and the sensor, wherein the sensor is configured to transmit a transmit signal into a field of view of the sensor, and generate

**EP 4 443 191 B1**

the receive signal based on received reflections of the transmitted transmit signal.

Some aspects of the present disclosure relate to an electronic device, comprising the above system as described herein, and control circuitry configured to control an operation of the electronic device based on the processed data.

Some aspects of the present disclosure relate to a computer-implemented method, comprising processing data indicating a receive signal of a sensor through isolating a locally stationary signal component of the receive signal from at least one of a stochastic and a deterministic signal component of the receive signal. The locally stationary signal component indicates a breathing motion in a field of view of the sensor. The processing of the data comprises selecting at least two frames from a plurality of frames of the data based on a predefined breathing rate assumed for the breathing motion and feeding the selected frames into a moving target indicator.

**Brief description of the Figures**

Some examples of apparatuses and/or methods will be described in the following by way of example only, and with reference to the accompanying figures, in which

Fig. 1 illustrates an example of an apparatus;

Fig. 2a and Fig. 2b illustrate examples of a diagram for a fast time dimension over a slow time dimension of an intermediate frequency signal;

Fig. 3a to Fig. 3d illustrate examples of a time diagram of frames of the intermediate frequency signal of the examples of Fig. 2a and Fig. 2b;

Fig. 4a and Fig. 4b illustrate examples of a correlation of slow time frames of the examples of Fig. 3a to Fig. 3d;

Fig. 5a and Fig. 5b illustrate examples of a correlation of fast time frames of the examples of Fig. 2a and Fig. 2b;

Fig. 6a and Fig. 6b illustrate an example of a range spectrum of two signal components of a receive signal of a radar sensor;

Fig. 7a and Fig. 7b illustrate an example of time-domain representation of a receive signal of a radar sensor;

Fig. 8 illustrates an example of an amplitude curve of a three frames which are fed into a moving target indicator and an output of the moving target indicator;

Fig. 9 illustrates an example of a plurality of consecutive frames of data indicating a receive signal of a sensor;

Fig. 10 illustrates another example of a plurality of consecutive frames of data indicating a receive signal of a sensor;

Fig. 11 illustrates an example of an output of 5 moving target indicator channels and a mean of the output;

Fig. 12 illustrates another example of an apparatus comprising a detector;

Figs. 13a to 13c illustrate an example of an output of a multi-channel moving target indicator for a first scenario;

Figs. 14a to 14c illustrate an example of an output of a multi-channel moving target indicator for a second scenario;

Figs. 15a to 15d illustrate an example of a range representation of a combined output of a multi-channel moving target indicator for a third scenario;

Fig. 16 illustrates an example of a sensor system;

Fig. 17 illustrates an example of an electronic device; and

Fig. 18 illustrates an example of a method.

**Detailed Description**

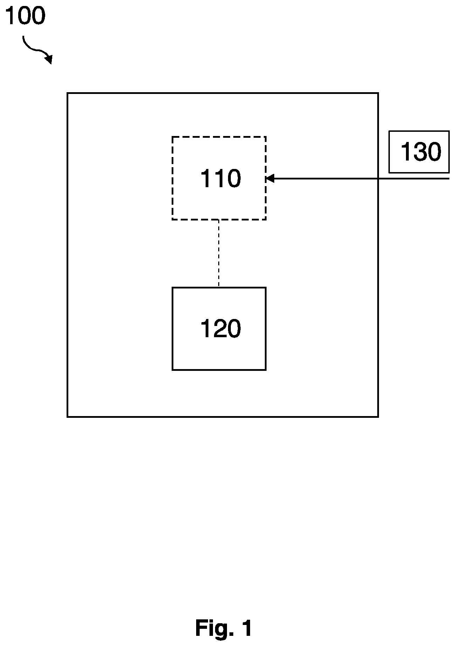

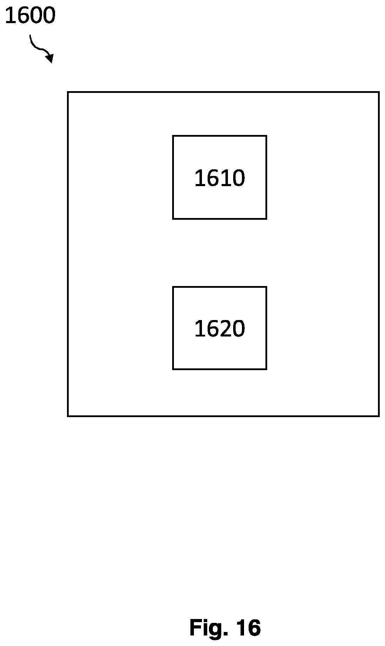

**Fig. 1** illustrates a block diagram of an example of an apparatus 100. For instance, the apparatus 100 may be integrated into a sensor system comprising a sensor such as explained below with reference to Fig. 16 or may be external to the sensor system. In the former case, the apparatus 100 may be (e.g., partially or fully) integrated into the sensor.

The apparatus 100 comprises optional interface circuitry 110 and processing circuitry 120. In case, interface circuitry 110 is present, the interface circuitry 110 may be communicatively coupled (e.g., via a wired or wireless connection) to the processing circuitry 120, e.g., for data exchange between the interface circuitry 110 and the processing circuitry 120.

The interface circuitry 110 may be any device or means for communicating or exchanging data. In case, the apparatus 100 comprises the interface circuitry 110, the interface circuitry 110 may be configured to receive (sensor) data 130 indicating a receive signal of the sensor. For instance, the interface circuitry 110 may be communicatively coupled to the sensor or to a storage device storing the data 130. The interface circuitry 110 may receive the data 130, e.g., via a wired or wireless coupling to the sensor or the storage device.

The receive signal may be any signal which is generated by the sensor based on received reflections of a transmit signal transmitted by the sensor into a field of view of the sensor. The sensor may, for instance, be a sensor for ranging, motion detection, presence detection or alike. The data 130 may indicate the receive signal in the sense that it encodes or represents the receive signal or a modified (e.g., noise-reduced or DC-removed (direct current) version thereof, e.g., modified in an upstream processing step performed by processing circuitry external to or integrated into the apparatus 100 (e.g., the processing circuitry 120). For instance, the data 130 may be or may be based on "raw data" of the sensor. In some examples, the receive signal is a receive signal of at least one of a radar sensor, a lidar sensor, an optical time-of-flight sensor, a sonar sensor and an ultrasonic sensor. Depending on the type of the sensor or on upstream preprocessing, the data 130 may, for instance, indicate a time-domain, a frequency-domain or a pulse-compressed version of the receive signal.

In other examples than the one shown in Fig. 1, the processing circuitry 120 may determine the data 130. In such cases, the apparatus 100 may dispense with the interface circuitry 110. For instance, the processing circuitry 120 may be integrated into the sensor and perform further processing of the data 130 within the sensor. The processing circuitry 120 may determine the data 130 by, e.g., sampling the receive signal and optionally modifying the sampled receive signal in a pre-processing step, e.g., for noise-reduction, DC-removal or alike. For instance, the apparatus 100 may comprise memory configured to store the determined data 130.

Alternatively, the processing circuitry 120 may partially determine the data 130. For instance, the processing circuitry 120 may determine a first part of the data 130, whereas at least one external processing circuitry may determine at least one second part of the data 130. The processing circuitry 120 and the external processing circuitry may, e.g., be connected within a distributed computing environment for jointly determining the data 130. In this case, the processing circuitry 120 may either be integrated into the sensor or may be external to the sensor. The processing circuitry 120 may receive the second part of the data 130, e.g., via an interface to the external processing circuitry such as interface circuitry 110, and further process the first and the second part of the data 130, as described below.

Alternatively, the processing circuitry 120 may be partially integrated into the sensor and be partially external to the sensor. For instance, the processing circuitry 120 may comprise a first part (first processing circuitry) which is integrated into the sensor and a second part (second processing circuitry) which is external to the sensor. In this case, the determination of the data 130 and/or further processing, as described below, may be performed by the first and second part of the processing circuitry 120 in a distributed manner.

The processing circuitry 120 may be, e.g., a single dedicated processor, a single shared processor, or a plurality of individual processors, some of which or all of which may be shared, a digital signal processor (DSP) hardware, an application specific integrated circuit (ASIC), a microcontroller or a field programmable gate array (FPGA). The processing circuitry 120 may optionally be coupled to, e.g., read only memory (ROM) for storing software, random access memory (RAM) and/or non-volatile memory.

The processing circuitry 120 is configured to process the data 130 through (by) isolating a locally stationary signal component of the receive signal from at least one of a stochastic and a deterministic signal component of the receive signal. For instance, the processing circuitry 120 may be configured to isolate the locally stationary signal component from the at least one of the stochastic and the deterministic signal component by extracting the locally stationary signal component of the receive signal. Alternatively or additionally, the processing circuitry 120 may be configured to isolate the locally stationary signal component from the at least one of the stochastic and the deterministic signal component by at least one of attenuating, filtering, suppressing and removing the at least one of the stochastic and the deterministic signal component of the receive signal. That is, the processing circuitry 120 may isolate the locally stationary signal component by either (directly) extracting (e.g., amplifying, determining or alike) the locally stationary signal component based on characteristics of the locally stationary signal component indicating local stationarity, or by (indirectly) extracting the locally stationary by excluding, attenuating or alike other non locally stationary signal components of the receive signal (the at least one of the stochastic and the deterministic signal component) based on characteristics of these signal components indicating randomness and determinism, respectively, or by a combination of both, indirect and direct means.

The processing circuitry 120 may, for instance, extract the locally stationary signal component by, e.g., detecting the locally stationary signal component, cutting or clipping the locally stationary signal component, filtering (e.g., by means of a (digital) filter) the locally stationary signal component, or amplifying (e.g., digitally or by means of an amplifier) the locally stationary signal component.

The processing circuitry 120 may, for instance, attenuate the at least one of a stochastic and a deterministic signal component by, e.g., filtering out or substantially removing the at least one of a stochastic and a deterministic signal component or by suppressing detection of a motion, presence or alike based on the at least one of a stochastic and a deterministic signal component. For instance, the processing circuitry 120 may differentiate between a locally stationary signal component and at least one of a stochastic and a deterministic signal component in the receive signal (based on the data 130). For instance, the processing circuitry 120 may determine whether (a part or component of) the receive signal indicates a locally stationary, a stochastic and/or a deterministic process (or motion) in the field of view of the sensor by classifying the receive signal (or the said part) as a locally stationary, a stochastic and/or a deterministic signal component.

A locally stationary signal component may be a part/component of the receive signal which exhibits local stationarity. Local stationarity may be ascribed to a non-stationary process which exhibits at least one statistical property changing slowly over time, e.g., (substantially) staying the same over a time of at least 2 or at least 4 seconds (on average).

Alternatively, local stationarity may be ascribed to a process which locally, in at least one or each sample (or time) point, is close to a stationary process (e.g., with a predefined relative error with respect to an approximated stationary process) but whose characteristics (a covar-iance, a parameter, or alike) are gradually changing, e.g., in a specific or an unspecific way. For instance, local stationarity may be modelled by a parameterized function whose parameter or coefficient is allowed to change smoothly over time. Examples of a locally stationary process may be a time-varying autoregressive process or a generalized autoregressive conditional heteroskedasticity (Garch) process.

An example of a nonparametric definition of local stationarity may be given by Equation 1:

$$Y_{t,T} = m\left(\frac{t}{T}, X_{t,T}\right) + \varepsilon_{t,T} \qquad \text{for } t = 1, \dots, T$$

Equation 1, with expectancy $E[\varepsilon_{t,T} | X_{t,T}] = 0$, where $Y_{t,T}$ and $X_{t,T}$ are random variables of dimension 1 and d, respectively. These variables may be assumed to be locally stationary, and their regression function may be allowed to change smoothly over time. The function m may be independent of real time t but dependent on a rescaled time t/T.

For example, (e.g., heuristically), a process $\{X_{t,T} : t = 1, \dots, T\}^\infty_{T=1}$ may be locally stationary if it behaves approximately stationary locally in time. An example of a rigorous definition of local stationarity may be to require that locally around each, at least one or a specific number of sample (or time) points u of the process $\{X_{t,T}\}$, the process $\{X_{t,T}\}$ is approximable by a stationary process $\{X_t(u) : t \in Z\}$ in a stochastic sense. For example, the process $\{X_{t,T}\}$ may be defined as locally stationary if for each rescaled sample point $u \in [0, 1]$ (e.g., rescaled to the unit interval according to an infill asymptotic approach) there exists an associated process $\{X_t(u)\}$ being strictly stationary with density $f_{X_t(u)}$ and meeting the following Equation 2:

$$\|X_{t,T} - X_t(u)\| \leq \left(\left|\frac{t}{T} - u\right| + \frac{1}{T}\right) U_{t,T}(u)$$

Equation 2, where $\{U_{t,T}(u)\}$ is a process of positive variables satisfying the expectancy $E[(U_{t,T}(u))^\rho] < C$ for some $\rho > 0$ and $C < \infty$ independent of u, t, and T. $\|\cdot\|$ denotes an arbitrary norm on real numbers with dimension d, $R^d$. The $\rho$-th moments of the variables $U_{t,T}(u)$ may be uniformly bounded. The constant $\rho$ may be regarded as a measure of how well $X_{t,T}$ is approximated by $X_t(u)$: For instance, the larger $\rho$ is chosen, the less mass is contained in the tails of the distribution of $U_{t,T}(u)$. Thus, if $\rho$ is large, then its bound $(|t/T - u| + 1/T) \cdot U_{t,T}(u)$ will take rather moderate values for most of the time, i.e., the bound and the approximation of $X_{t,T}$ by $X_t(u)$ is stronger and more accurate, respectively, for larger $\rho$.

The locally stationary signal component indicates a breathing motion in the field of view of the sensor, e.g., a breathing motion (due to respiratory activity) of a living being such as a human or an animal. A breathing motion may exhibit a local stationarity in a sense that it causes a regular, deterministic or periodic pattern (such as continuous inhale and consecutive exhale intervals) in the receive signal which has a statistical property (such as the (mean) time of a breathing cycle (breathing rate), the (mean) time of the inhale and exhale intervals, or the (mean) range or depth in which the breathing motion takes place) that changes slowly over time, e.g., staying constant (or not changing significantly) over at least two breathing cycles or over at least 8 seconds.

For example, assuming that an adult person at rest has a constant breathing rate (e.g., 4 seconds per breath), the intermediate frequency signal may experience a substantially constant phase shift (between frames of the data 130) during inhale and exhale cycles. Hence, the locally stationary signal component may remain stationary for, e.g., at least 2 seconds, at least within one inhale or exhale phase (interval). If the inhale and the exhale phase exhibit the same rate, then the locally stationary signal component may remain stationary for, e.g., at least 4 seconds. Even if the phase of the locally stationary signal component, e.g., induced by micro-Doppler, may change its sign, it may still remain stationary, since it's autocorrelation function may depend on the lag only. When the breathing rate of a person at rest is assumed to vary within 3 to 5 seconds, an interval of 1,5 to 2,5 seconds may be the minimum time frame in which the signal component remains stationary.

A deterministic signal (e.g., (globally) stationary) component may be a part/component of the receive signal which exhibits a deterministic, e.g., regular, nature. For example, a value of the deterministic signal component may be determinable without uncertainty, e.g., at any instant of time. For example, the deterministic signal component may be definable by a mathematical formula, e.g., with no or non-varying parameters. A deterministic signal component may, e.g., indicate a regular motion or a vibration in the field of view of the sensor such as a motion of a fan.

A stochastic (e.g., random) signal component may be a part/component of the receive signal which varies in a random manner or exhibits a random variable. For instance, the stochastic signal component may indicate a random motion in the field of view of the sensor such as a motion of a curtain moving in the wind.

In some examples, the at least one of the stochastic and the deterministic signal component indicates at least one of a random motion and a vibration in the field of view of the sensor. For instance, the at least one of the stochastic and the deterministic signal component may indicate a motion of a curtain or a fan in the field of view of the sensor.

For instance, if the receive signal and, thus, the locally stationary signal component is an intermediate frequency signal $x_{IF}(t)$ of a radar sensor, $x_{IF}(t)$ may be defined for an FMCW (Frequency Modulated Continuous Wave) signal by Equation 3:

$$x_{IF}(t) = \cos\left(2\pi \frac{2R}{c}\mu t_0 \mp 2\pi \frac{2v}{\lambda_c}T_{ich}\right)$$

Equation 3, where $\mu$ is the chirp rate, R is the range of a target, $v$ is the velocity of the target, $T_{ich}$ is the inter-chirp interval and $t_0$ is the duration of the chirp. $x_{IF}(t)$ (of Equation 3) can also be stated in complex form (Equation 4) for illustrative purposes (analysis convenience). A single-sided spectrum may be considered in the following, i.e., as if a Hilbert transform had been applied or as if a receiver of the radar sensor had employed an In-phase-Quadrature (I/Q) circuitry. In other examples, a two-sided spectrum may be considered.

In the case of a cosine-modulated random process, the intermediate frequency signal may be defined by Equation 4:

$$x_{IF}(t) = exp\left(j2\pi \frac{2R}{c}\mu t_0\right) \cdot exp\left(j \mp 2\pi \frac{2v}{\lambda_c}T_{ich}\right) = a(t)\{u(t)\}$$

Equation 4, where a(t) is a deterministic function and {u(t)} is a stationary random process with a mean and a variance of zero and unity, respectively.

Equation 4 may satisfy the definition of a locally stationary process according to Equation 5:

$$\mathcal{R}_{xx}(\tau, t) \approx \mathcal{R}_{xx}(0, t)\mathcal{R}_{uu}(\tau) = \psi_x^2(t)\mathcal{R}_{uu}(\tau)$$

Equation 5, where $R_{xx}(\tau,t)$ is the correlation of $x_{IF}(t)$, where $\mathcal{R}_{xx}(0,t)$ is the instantaneous mean square value of $\{x_{IF}(t)\}$, and $\mathcal{R}_{xx}(0, t) = \psi_x^2(t) = a^2(t)$ holds since $a(t) = exp\left(j2\pi \frac{2R}{c}\mu t_0\right)$ is a completely deterministic function, and where $R_{uu}(\tau)$ is the stationary correlation function of {u(t)}, and $\mathcal{R}_{uu}(\tau) = E\left[u\left(t - \frac{\tau}{2}\right)u\left(t - \frac{\tau}{2}\right)\right]$ holds for as long as {u(t)} (i.e., velocity or breathing rate) remains constant (i.e., $R_{uu}(\tau)$ may depend only on the lag $\tau$, but not on the time instance t).

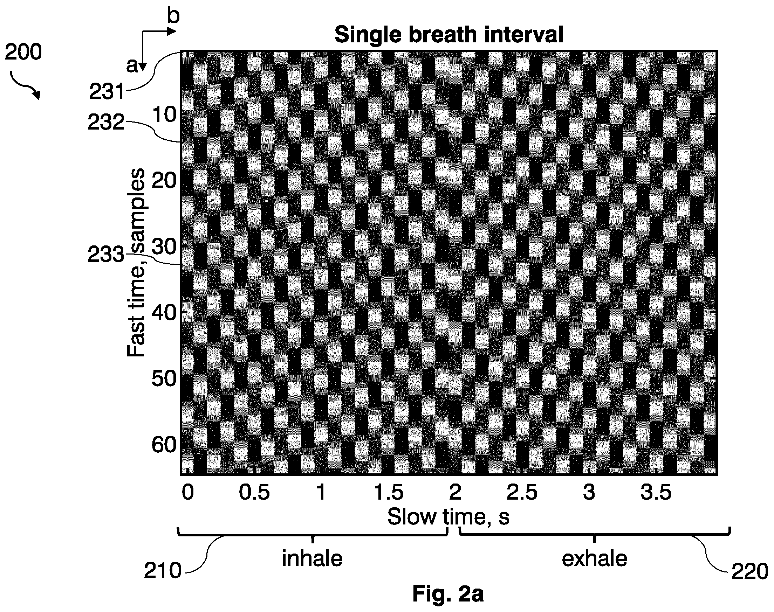

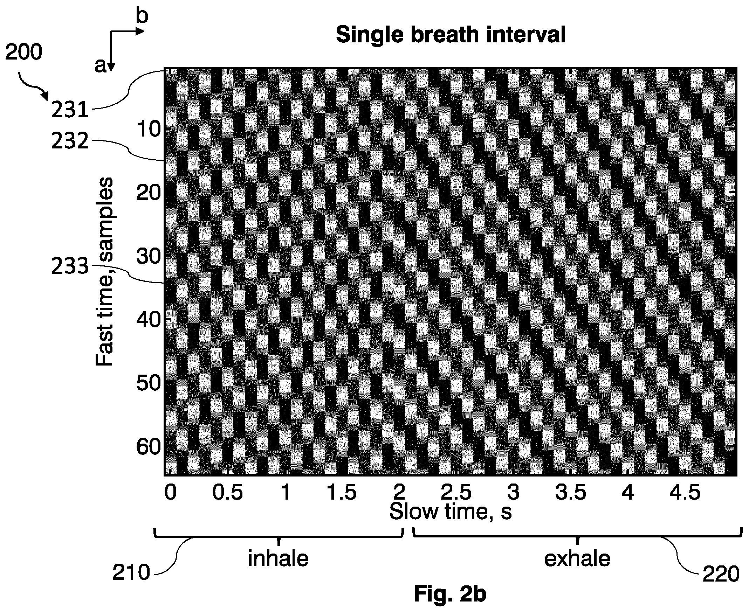

Examples of an intermediate frequency signal of a radar sensor are illustrated by **Fig. 2a** and **Fig. 2b** which show example of a diagram 200 for a fast time dimension a over a slow time dimension b of the intermediate frequency signal. The intermediate frequency signal comprises a locally stationary signal component in the examples of Fig. 2a and Fig. 2b. The diagram 200 refers to a scenario where a breathing motion is present in a field of view of the radar sensor. The diagram 200 shows samples (sorted by time) of individual chirps along the fast time dimension a and the individual chirps (sorted by time) along the slow time dimension b. In Fig. 2a and Fig. 2b, the diagram 200 shows 64 samples in the fast time dimension a and 4 and 5 seconds, respectively, in the slow time dimension b for illustrative purposes. The samples within one row over slow time dimension b may be a fast time frame, e.g., fast time frame 231 with the (row) index 1, fast time frame 232 with the index 15 and fast time frame 233 with the index 34. The samples within one column over fast time dimension a may be a slow time frame.

The samples of Fig. 2a and Fig. 2b show a first regular pattern 210 over a time span from 0 to 2 seconds which corresponds to an inhale interval of the breathing motion and a second regular pattern 220 over a time span from 2 to 4 and from 2 to 5 seconds, respectively, which corresponds to an exhale interval of the breathing motion. The two regular patterns 210, 220 form a discontinuation at second 2 since the inhale and the exhale motions have differing motion patterns (e.g., have opposing directions).

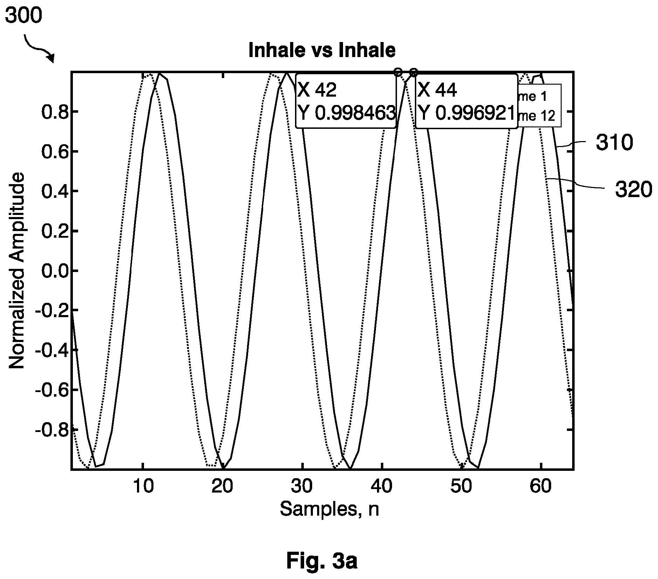

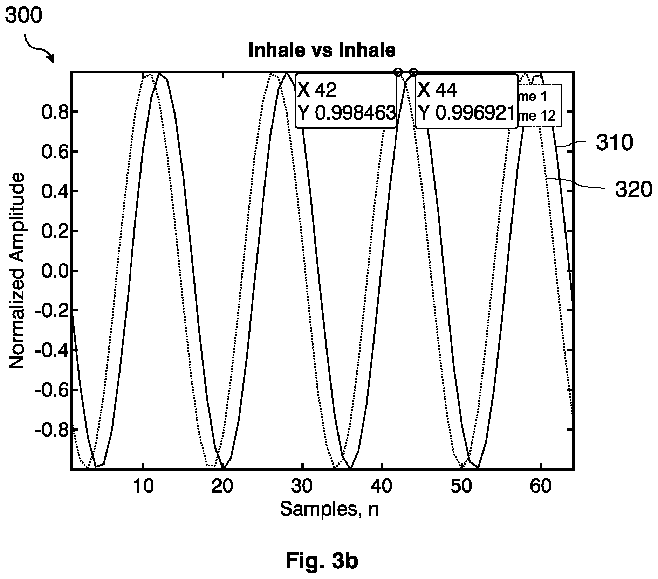

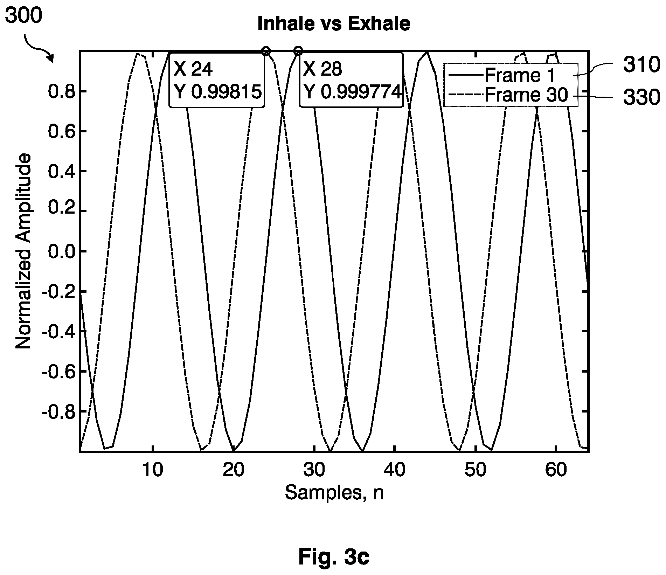

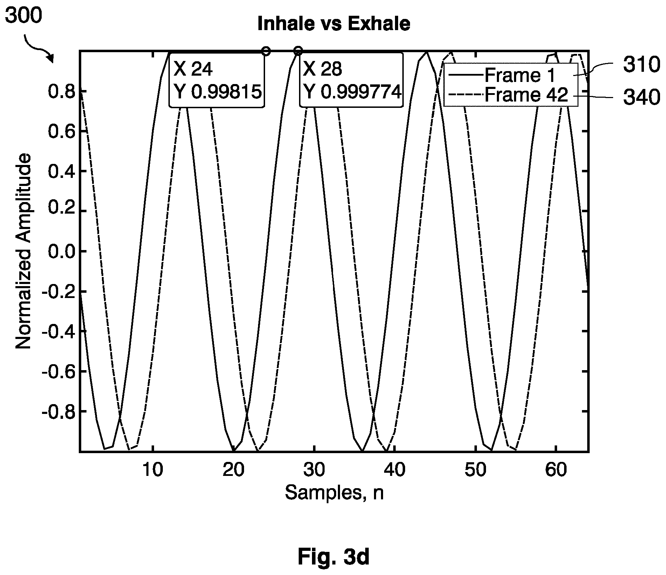

**Fig. 3a** to **Fig. 3d** illustrate examples of a time diagram 300 for the intermediate frequency signal of the example of Fig. 2a and Fig. 2b. Fig. 3a and Fig. 3c relate to the example of Fig. 2a, while Fig. 3b and Fig. 3d relate to the example of Fig. 2b.

The time diagram 300 of Fig. 3a and Fig. 3c shows a normalized amplitude of chirps of a first slow time frame 310 (i.e., a certain selection of chirps in slow time), here frame with index 1, over fast time for the example of Fig. 2a. The time diagram 300 of Fig. 3c and Fig. 3d shows a normalized amplitude of chirps of a first slow time frame 310 (i.e., a certain selection of chirps in slow time), here frame with index 1, over fast time for the example of Fig. 2b.

Further, the time diagram 300 of Fig. 3a and Fig. 3c shows a normalized amplitude of chirps of a second slow time frame 320 (frame with the (column) index 12) and a third slow time frame 330 (frame with the index 30) of the example of Fig. 2a. The time diagram 300 of Fig. 3b and Fig. 3d shows a normalized amplitude of chirps of the third slow time frame 330 and fourth slow time frame 340 (frame with the index 42) of the example of Fig. 2b. The frames 310 to 340 show each a periodic amplitude over time.

The first frame 310 of Fig. 3a and Fig. 3c is within the inhale interval of Fig. 2a. The first frame 310 of Fig. 3b and Fig. 3d is within the inhale interval of Fig. 2b. Thus, the first frame 310 is within the first regular pattern 210. The second frame 320 and the third frame 330 of

Fig. 3a and Fig. 3c are within the exhale interval of Fig. 2a, The second frame 230 and the fourth frame 340 of Fig. 3b and Fig. 3d are within the exhale interval of Fig. 2b. Thus, the frames 320 to 340 are within the second regular pattern 220. The frames 310 and 320, the frames 310 and 330, and the frames 310 and 340 exhibit a respective phase difference (time lag $\tau$) between each other reflecting the differing motion patterns of the inhale and the exhale motion.

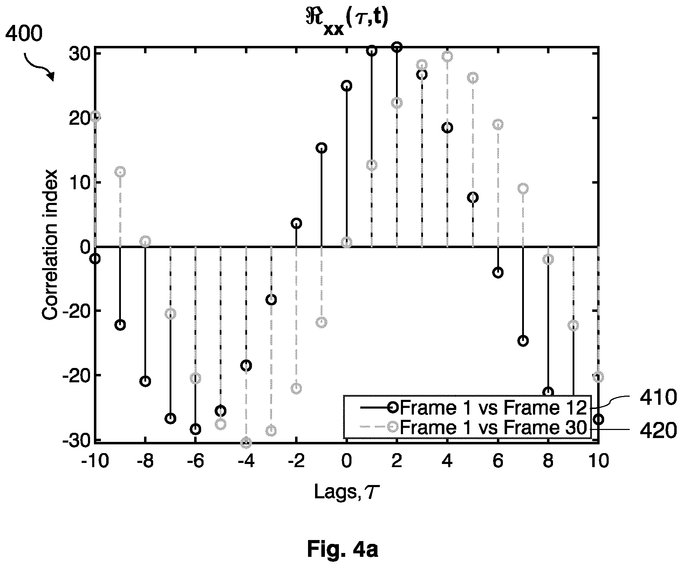

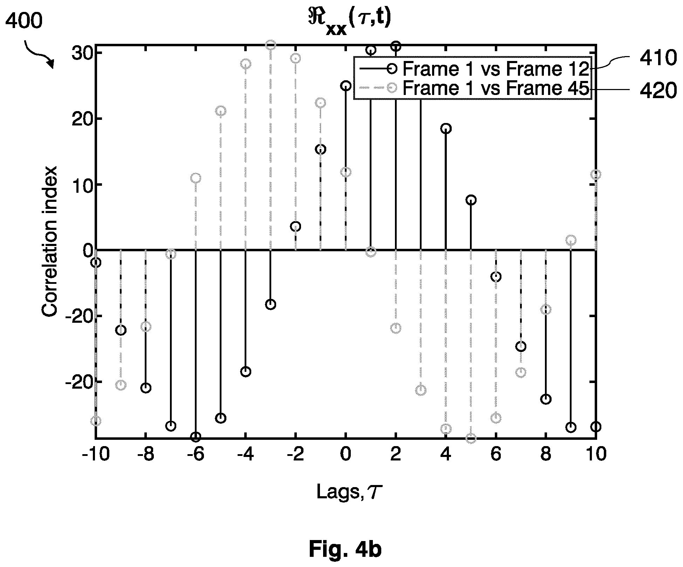

**Fig. 4a** illustrates an example of a correlation 400 for the examples of frames 310, 320, 330 of Fig. 3a and Fig. 3c. **Fig. 4b** illustrates an example of a correlation 400 for the example of frames 310, 320, 340 of Fig. 3b and Fig. 3d. In Fig. 4a, the correlation 400 is illustrated as first correlation index values 410 over time lag $\tau$ for frame 310 versus frame 320 and second correlation index values 420 over time lag $\tau$ for frame 310 versus frame 330. In Fig. 4b, the correlation 400 is illustrated as first correlation index values 410 over time lag $\tau$ for frame 310 versus frame 320 and second correlation index values 420 over time lag $\tau$ for frame 310 versus frame 340. The first correlation index values 410 and the second correlation index values 420 exhibit periodic patterns over time lag $\tau$ which are phase-shifted with respect to each other.

The correlation 400 of the examples of Fig 4a and Fig. 4b may be defined by the following Equation 6 satisfying the statement of Equation 7:

$$\mathcal{R}_{xx}(\tau, t) \approx \mathcal{R}_{xx}(0, t)\mathcal{R}_{uu}(\tau) = \psi_x^2(t)\mathcal{R}_{uu}(\tau)$$

Equation 6

$$\mathcal{R}_{uu}(-\tau) \neq \mathcal{R}_{uu}(\tau)$$

Equation 7

Equation 7 shows that the correlations (correlation coefficients) 410 and 420 may be constant at different instances of time (t), but they depend on the lag ($\tau$). This may indicate local stationarity. (Global) stationarity would be indicated by fulfilment of the statement of Equation 8:

$$\mathcal{R}_{uu}(-\tau) = \mathcal{R}_{uu}(\tau)$$

Equation 8

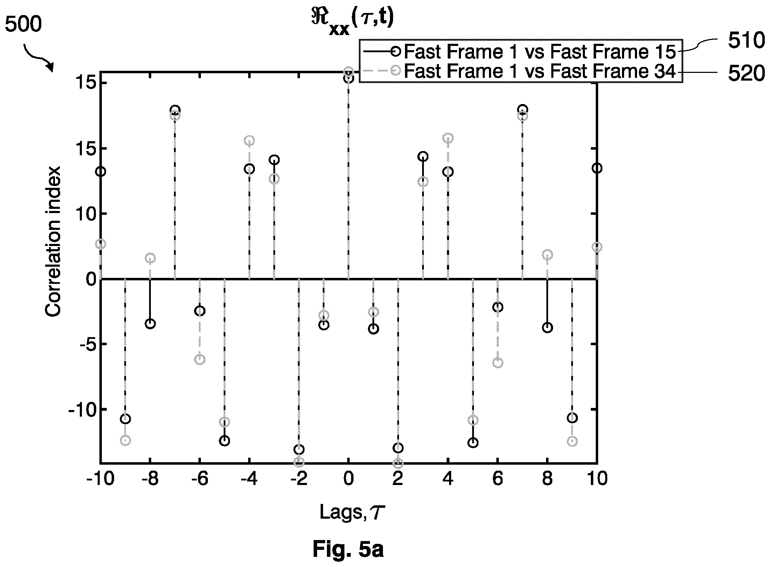

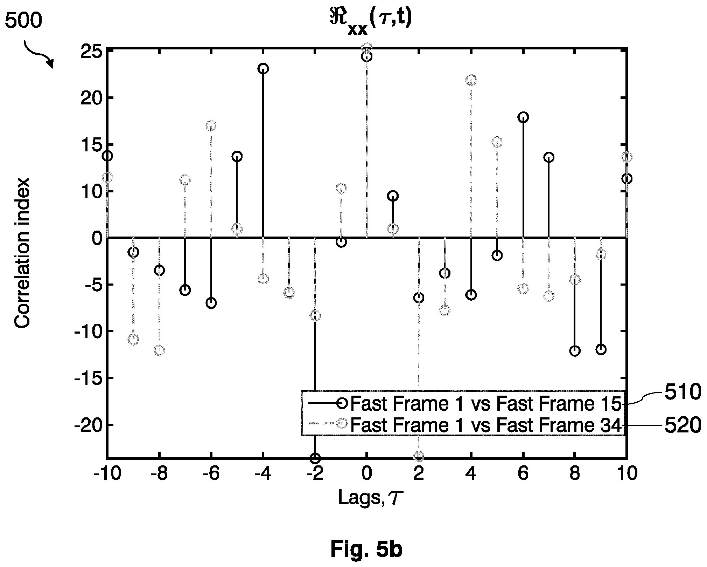

However, if the two intervals - inhale interval 210 and exhale interval 220 - were considered separately, these intervals would be stationary. **Fig. 5a** and **Fig. 5b** illustrate examples of a correlation 500 of the fast time frames 231, 232, 233 of the diagram 200 of Fig. 2a and Fig. 2b, respectively. The correlation 500 is illustrated as first correlation index values 510 over time lag $\tau$ for frame 231 versus frame 232 and second correlation index values 520 over time lag $\tau$ for frame 231 versus frame 233. In the example of Fig. 5a and Fig. 5b, the correlation 500 would fulfill Equation 8 and would therefore indicate stationarity.

Thus, frames of the data 130 along the fast time dimension (axis) may satisfy the local stationarity criterion, while frames of the data 130 along the slow time dimension (axis) may satisfy the stationarity criterion, e.g., as long as there is no change in the breathing rate.

Alternatively, in some examples, the locally stationary signal component indicates a heartbeat in the field of view of the sensor.

Depending on the chosen method for isolating the locally stationary signal component, the data 130 may be required to have a certain structure. For example, the processing circuitry 120 may be configured to process the data 130 in a time-domain or a frequency-domain of the receive signal. This may require, in some cases, a pre-processing of the data 130. For instance, if the sensor is a radar sensor such as an FMCW radar, the data 130 may be initially provided in a time-domain of the receive signal. For instance, the FMCW radar may perform stretch processing on the receive signal which generates the data 130 in time-domain. The processing circuitry 120 may, e.g., process the data 130 in time-domain or transform the time-domain version of the receive signal into the frequency-domain (into a range spectrum), e.g., by performing a (e.g., fast) Fourier or Laplace transformation on the data 130, for continuing with frequency-domain processing of the transformed data 130.

If the sensor is an optical time-of-flight sensor, a sonar sensor or an ultrasonic sensor, the receive signal may encode pulsed waveforms. The data 130 may be initially provided in frequency-domain, e.g., if a pulse compression technique is applied to the pulsed waveforms and the frequency-domain (range spectrum) version of the receive signal is generated by correlation. Alternatively, the processing circuitry 120 may transform the pulsed waveforms into the frequency-domain based on a correlation technique. In both cases, the processing circuitry 120 may, e.g., process the data 130 in the frequency-domain or convert the frequency-domain version to time-domain, e.g., by performing an IFFT (inverse fast Fourier transformation) on the frequency-domain data.

Further, e.g., depending on the resolution provided by the sensor and the motion causing the locally stationary signal, the locally stationary signal component may be a micro-Doppler signal component or a macro-Doppler signal component of the receive signal. For instance, a target with a locally stationary motion in the field of view of the sensor may cause the locally stationary signal component. The locally stationary motion may be a small motion which induces additional phase shift, e.g., proportional to the target's velocity, on the signal returns (reflections of the transmitted signal). This phase shift may be a micro-Doppler signal component. In the case of a micro-Doppler signal component, a time-domain processing of the data 130 may be beneficial since frequency-domain processing may mitigate micro-Doppler signal components.

For instance, the resolution of a 24 GHz (gigahertz) radar may be too small to directly detect a breathing motion such as the movement of a chest of a human in the field of view of the radar sensor, i.e., no measurable change may be introduced to the frequency of the radar return (reflections of the transmitted signal). The breathing motion may still be detectable through monitoring an instantaneous phase of the radar return (e.g., of an intermediate frequency signal), i.e., a micro-Doppler signal component or signature in the receive signal.

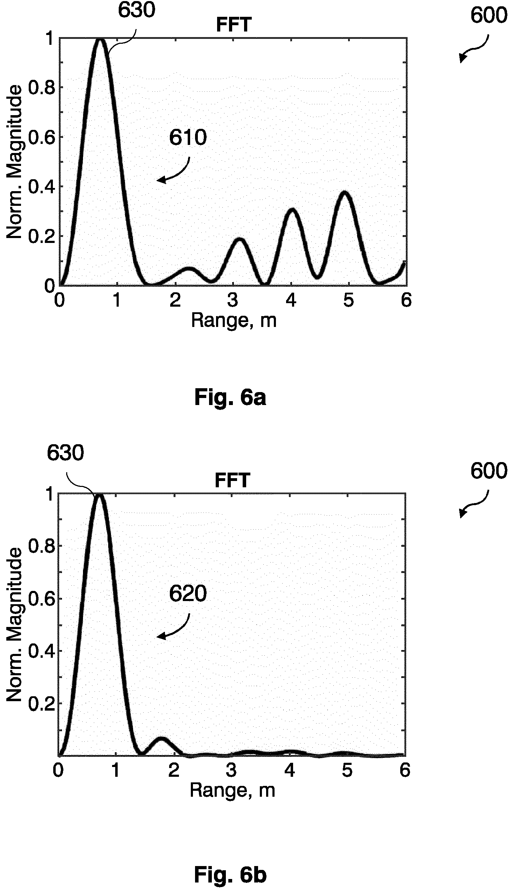

The latter is illustrated by **Fig. 6a and Fig. 6b** which show an example of a range spectrum 600 of two signal components of a receive signal of a radar sensor. The range spectrum 600 shows a normalized signal strength of the two signal components over a range (corresponding to a frequency of the two signal components). The two signal components correspond to an exhale interval and an inhale interval of a breathing motion, respectively, yielding a first curve 610 and a second curve 620 of the range spectrum 600. For example, a static person may be located 0,75 meters away from a 24 GHz radar sensor (bandwidth 200 MHz; megahertz). The static person may be represented in the receive signal of the radar sensor as a sine-wave of fixed frequency corresponding to 0,75 meters range (e.g., one complete cycle of the sine-wave). Therefore, in Fig. 6a and Fig. 6b, both the curves 610 and 620 of the range spectrum 600 exhibit a peak 630 at a range of 0,75 meters. The motion of the chest of the static person may barely be reflected in the range spectrum 600 of the sine-wave. Thus, the breathing motion may be difficult to detect in the macro-Doppler signal component.

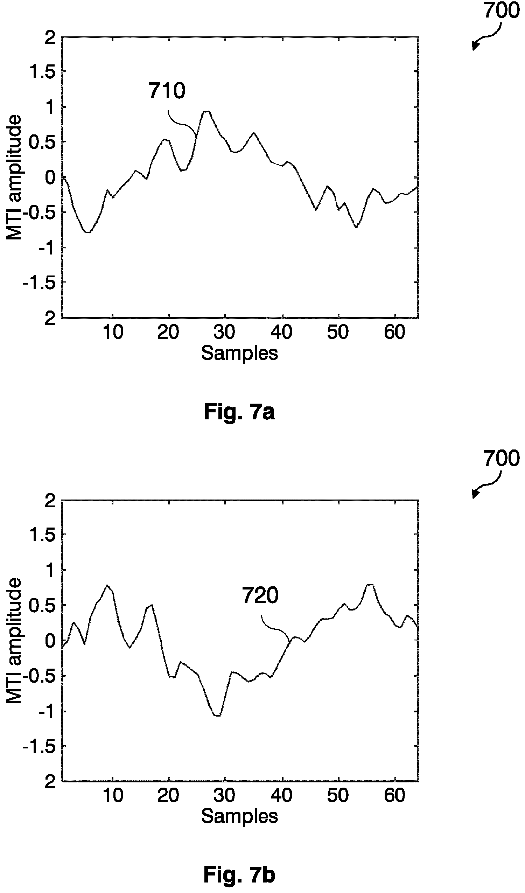

However, the breathing motion may be visible in the micro-Doppler signal. For instance, **Fig. 7a and Fig. 7b** illustrate an example of time-domain representation 700 of an example of a receive signal showing an amplitude curve of the receive signal over time. Fig. 7a and Fig. 7b illustrate a first sine wave 710 and a second sine wave 720 in a receive signal of a radar sensor corresponding to the exhale and the inhale interval of Fig. 6a and Fig. 6b, respectively. The two sine waves 710 and 720 may represent a respective signal component of an intermediate frequency signal (of the receive signal) of the radar sensor. They exhibit a phase shift relative to each other of about 180 degrees but may exhibit the same frequency. Therefore, a breathing motion may be detectable based on its micro-Doppler signature whereas its macro-Doppler signature may completely hide it. Thus, the use of micro-Doppler signatures may be beneficial for such cases.

Alternatively, the locally stationary signal component may be a macro-Doppler signal component, e.g., a signal component measurable within the resolution of the sensor. For example, a radar sensor with a higher bandwidth (1 GHz or above) may provide a macro-Doppler signature of a breathing motion in the receive signal.

In a concrete example, the locally stationary signal component may be an intermediate frequency (IF) signal $x_{IF}$(t). $x_{IF}$(t) may be determined based on a transmit signal $x_t$(t) transmitted by the sensor and a received reflection $x_r$(t) of the transmit signal. In case of an FMCW radar sensor employing a linear chirp, $x_t$(t) and $x_r$(t) may be modelled according to Equation 9 and Equation 10, respectively:

$$x_t(t) = \cos(2\pi f_c t_0 + \pi \mu t_0^2)$$

Equation 9, where $f_c$ is the center frequency, $t_0$ the duration of the chirp, $\mu = BW/t_0$ is the chirp rate, where $BW$ is its bandwidth.

$$x_r(t) = A\cos(2\pi f_c(t_0 - \Delta\tau) + \pi\mu(t_0 - \Delta\tau)^2)$$

Equation 10, where $\Delta\tau$ is a parameter characterizing the delay between transmission and reception associated with the round trip to the target and, in case of the moving target, characterizing a Doppler shift; A is the amplitude of the return signal (reflection).

The delay $\Delta\tau$ may be described by Equation 11:

$$\Delta\tau = \frac{2R}{c} \mp \frac{2v}{c}$$

Equation 11, where the first term is associated with the round trip to the object, the second term is the Doppler shift (- sign for approaching object and + sign for receding target, $v$ is the target's velocity and c is the speed of light).

The intermediate frequency signal $x_{IF}$(t) may be described by Equation 12 (e.g., after mixing $x_t$(t) and $x_r$(t)) and lowpass filtering):

$$x_{IF}(t) = \cos(2\pi\mu\Delta\tau t_0 + 2\pi f_c\tau - \pi\mu\Delta\tau^2)$$

Equation 12, where the last term ($\pi\mu\Delta\tau^2$) may be ignored when $\Delta\tau^2 \to 0$, where the first term ($2\pi\mu\Delta\tau t_0$) has time-dependency and defines the instantaneous frequency $f_{inst}$ of the IF signal, the middle term ($2\pi f_c\tau$) is the instantaneous phase of the signal.

The instantaneous frequency may be written as per Equation 13:

$$f_{inst} = \frac{1}{2\pi}\frac{d}{dt}(2\pi\mu\Delta\tau t_0 + 2\pi f_c\tau) = \mu\Delta\tau = \frac{BW}{t_0}\left(\frac{2R}{c} \mp \frac{2v}{c}t_0\right) = \frac{2BWR}{ct_0} \mp \frac{2v}{c}BW$$

Equation 13, where, if the target's velocity is big enough, the second term ($\frac{2v}{c}BW$) is the macro-Doppler signal component, else the second term can be neglected ($\frac{2v}{c}BW \approx 0$), where, if $v \neq 0$, i.e., the object has non-zero speed (like in case with a breathing motion), the Doppler effect may manifest itself via an instantaneous phase of the IF signal ($2\pi f_c\tau$), i.e. a micro-Doppler signal component. A phase of the micro-Doppler signal component may be defined by Equation 14:

$$\varphi = 2\pi f_c\tau = 2\pi\left(\frac{2R}{c}f_c \mp \frac{2v}{c}t_0 f_c\right) = 2\pi\left(\frac{2R}{\lambda_c} \mp \frac{2v}{\lambda_c}t_0\right)$$

Equation 14, where the first term ( $\frac{2R}{\lambda_c}$ ) is a constant phase offset, related to the range of an object, which may be neglectable (e.g., a sitting person may substantially exhibit no motion, hence, $\frac{2R}{\lambda_c} = const$ ), where the second term may show that for as long as the velocity remains constant each subsequent frame will be phase shifted with respect to the previous one by $2\pi \frac{2v}{\lambda_c}$ . The second term may be rewritten as follows in Equation 15:

$$\varphi = 2\pi \frac{2v}{\lambda_c} T_{ich}$$

Equation 15, where $T_{ich}$ is the inter-chirp interval which may be equal to the frame rate, or it may be longer than the frame rate as in the case of a slow-rate moving target indicator (as explained below), where some frames may be deliberately skipped in individual channels of the moving target indicator.

This may result in the intermediate frequency signal $x_{IF}(t)$ being derived by Equation 16:

$$x_{IF}(t) = \cos\left(2\pi \frac{2R}{c} \mu t_0 \mp 2\pi \frac{2v}{\lambda_c} T_{ich}\right)$$

Equation 16, where R is the range defined by the frequency of the sine-waves (e.g., all frames may contain a signal of the same frequency), where velocity *v* may define the instantaneous phase of the sine-waves (sine-waves may be phase shifted in different frames).

Examples of how the data 130 is processed in order to extract the locally stationary signal component and attenuate the at least one of a stochastic and a deterministic signal component of the receive signal are given in the following: The processing circuitry 120 may, for example, process the data 130 by determining a (e.g., auto-) correlation between certain selected frames of the data 130, e.g., fast time frames and/or slow time frames of the data 130. The processing circuitry 120 may determine whether the receive signal comprises a locally stationary signal component by determining whether a stationary correlation between the selected frames is time-independent and/or whether the stationary correlation is dependent on a phase or time difference between the frames, e.g., based on Equation 7. Further, the processing circuitry 120 may be configured to process the data 130 through using a moving target indicator and optionally at least one of a linear predictor and a trained machine-learning model.

In case of a linear predictor, a linear relationship between a dependent variable, e.g., local stationarity, and an independent variable, e.g., a characteristic of a certain number of samples of the data 130 such as a distribution of values (of the samples) over a certain time period. This linear relationship may be defined as a function or linear combination of the independent variable (explanatory variable). E.g., by means of predefined coefficients (or weights), the processing circuitry 120 may determine whether local stationarity is present in the considered signal section of the receive signal based on the linear relationship and the independent variable. Examples of a linear predictor are a linear regression or a linear classifier such as linear discriminant analysis.

In case of the trained machine-learning model, the processing circuitry 120 may determine the local stationary signal component and the at least one of the stochastic and the deterministic signal component of the receive signal based on the trained machine-learning model, and extract or attenuate the determined local stationary signal component and the determined at least one of the stochastic and the deterministic signal component, respectively. The machine-learning model is a data structure and/or set of rules representing a statistical model that the processing circuitry 120 uses to perform the determination, extraction or attenuation without using explicit instructions, instead relying on models and inference. The data structure and/or set of rules represents learned knowledge (e.g. based on training performed by a machine-learning algorithm). For example, in machine-learning, instead of a rule-based transformation of data, a transformation of data may be used, that is inferred from an analysis of historical and/or training data. In the proposed technique, the content of the data 130 is analyzed using the machine-learning model (i.e., a data structure and/or set of rules representing the model).

The machine-learning model is trained by a machine-learning algorithm. The term "machine-learning algorithm" denotes a set of instructions that are used to create, train or use a machine-learning model. For the machine-learning model to analyze the content of data 130, the machine-learning model may be trained using training and/or historical data as input and training content information (e.g. labels indicating whether local stationarity, stationarity or randomness is present in a certain signal section in the data) as output. By training the machine-learning model with a large set of training data and associated training content information (e.g. labels or annotations), the machine-learning model "learns" to recognize the content of the data, so the content of data, such as the data 130, that are not included in the training data can be recognized using the machine-learning model. By training the machine-learning model using training data and a desired output, the machine-learning model "learns" a transformation between the data and the output, which can be used to provide an output based on non-training data provided to the machine-learning model.

The machine-learning model may be trained using training input data (e.g. training sensor data). For example, the machine-learning model may be trained using a training method called "supervised learning". In supervised learning, the machine-learning model is trained using a plurality of training samples, wherein each sample may comprise a plurality of input data values, and a plurality of desired output values, i.e., each training sample is associated with a desired output value. By specifying both training samples and desired output values, the machine-learning model "learns" which output value to provide based on an input sample that is similar to the samples provided during the training. For example, a training sample may comprise training sensor data as input data and one or more labels as desired output data.

Apart from supervised learning, semi-supervised learning may be used. In semi-supervised learning, some of the training samples lack a corresponding desired output value. Supervised learning may be based on a supervised learning algorithm (e.g., a classification algorithm or a similarity learning algorithm). Classification algorithms may be used as the desired outputs of the trained machine-learning model are restricted to a limited set of values (categorical variables), i.e., the input is classified to one of the limited set of values (type of exercise, execution quality). Similarity learning algorithms are similar to classification algorithms but are based on learning from examples using a similarity function that measures how similar or related two objects are.

Apart from supervised or semi-supervised learning, unsupervised learning may be used to train the machine-learning model. In unsupervised learning, (only) input data are supplied and an unsupervised learning algorithm is used to find structure in the input data such as training and/or historical sensor data (e.g. by grouping or clustering the input data, finding commonalities in the data). Clustering is the assignment of input data comprising a plurality of input values into subsets (clusters) so that input values within the same cluster are similar according to one or more (predefined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such that the cumulative reward is increased, leading to software agents that become better at the task they are given (as evidenced by increasing rewards).

Furthermore, additional techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e., outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g., a set of input sensor data) may be represented by the branches of the decision tree, and an output value corresponding to the item may be represented by the leaves of the decision tree. Decision trees support discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may, e.g., be used to store, manipulate or apply the knowledge.

For example, the machine-learning model may be an Artificial Neural Network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receive input values (e.g. the data 130), hidden nodes that are (only) connected to other nodes, and output nodes that provide output values (e.g., a flag indicating whether a locally stationary signal component is present). Each node may represent an artificial neuron. Each edge may transmit information from one node to another. The output of a node may be defined as a (non-linear) function of its inputs (e.g., of the sum of its inputs). The inputs of a node may be used in the function based on a "weight" of the edge or of the node that provides the input. The weight of nodes and/or of edges may be adjusted in the learning process. In other words, the training of an ANN may comprise adjusting the weights of the nodes and/or edges of the ANN, i.e., to achieve a desired output for a given input.

Alternatively, the machine-learning model may be a support vector machine, a random forest model or a gradient boosting model. Support vector machines (i.e., support vector networks) are supervised learning models with associated learning algorithms that may be used to analyze data (e.g., in classification or regression analysis). Support vector machines may be trained by providing an input with a plurality of training input values (e.g. sensor data) that belong to one of two categories (e.g., local stationarity, stationarity or randomness). The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection. In some example, the machine-learning model may be a combination of the above examples.

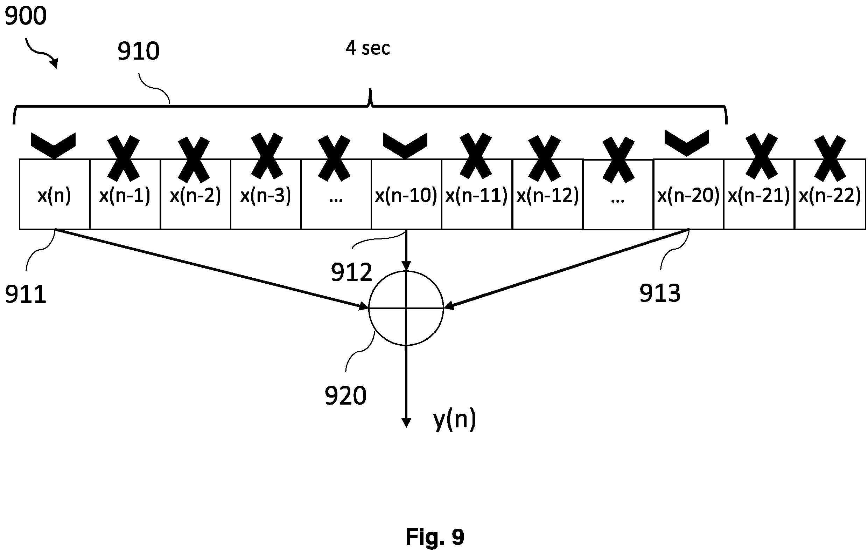

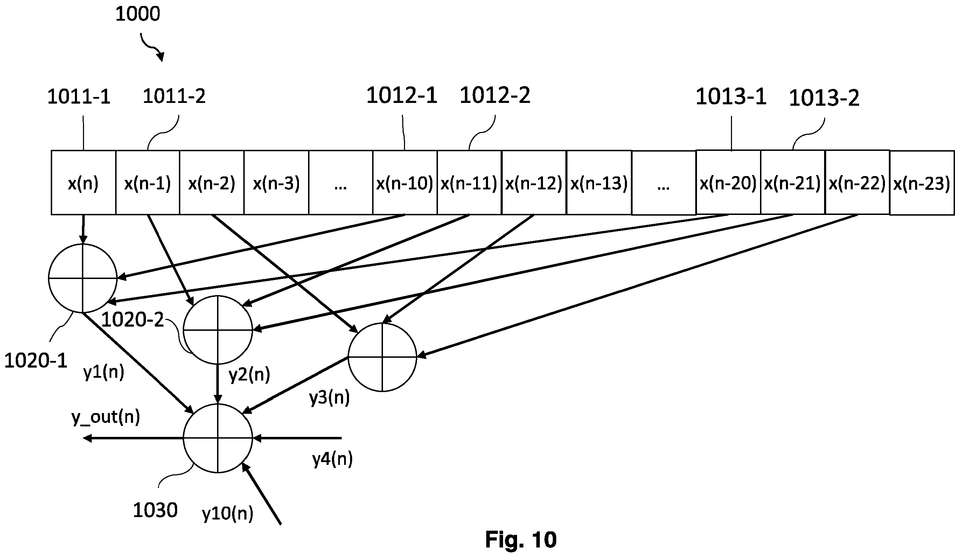

The processing circuitry 120 may filter the data 130 for extracting or attenuating signal components based on the moving target indicator. For instance, the processing circuitry 120 may filter the data 130 by using an MTI including at least one of a delay line canceller, a recursive filter or a bandpass filter. In case of a bandpass filter, the processing circuitry 120 may filter parts of the receive signal which do not show a frequency shift, thereby directly extracting moving targets, and analyze the motion of the moving targets. In case of the delay line canceller, the MTI may be at least a single delay canceller, and the processing circuitry 120 may provide a time delay corresponding to the pulse repetition interval of the sensor or a multiple thereof. The processing circuitry 120 may feed a frame of the data 130 (e.g., indicating a signal component of the receive signal) and a reference frame of the data 130 (e.g., indicating another signal component of the receive signal) into a summing node such as a phase detector whose output is proportional to the phase difference of the two inputs. The summing node may further combine the inputs such that a locally stationary signal component is extracted or amplified and a random or stationary signal component is attenuated. Examples of a configuration of the MTI are explained below and with reference to Figs. 9 and 10.

The processing circuitry 120 is configured to process the data 130 based on a predefined breathing rate assumed for the breathing motion. For example, the predefined breathing rate may be based on (e.g., a multiple of) a mean breathing rate, e.g., of one breathing cycle per 4 seconds. An average respiratory rate for adults may be about 15 breaths per minute, i.e., the corresponding signal component may repeat itself every 4 seconds but slowly changes. Therefore, an example of a predefined breathing rate may be chosen as 8 seconds. The latter may provide sufficient accuracy to determine local stationarity and on the other hand may enable a quick determination of the local stationarity in few seconds.

The processing circuitry 120 uses the MTI and optionally one or more of the above-mentioned techniques (linear predictor, trained machine-learning model) based on the predefined breathing rate. For example, the processing circuitry 120 may detect a periodic pattern in the receive signal with an average repetition frequency lying within a certain range around the predefined breathing rate. The processing circuitry 120 may then determine whether the periodic pattern changes and, optionally, a roughness at which it changes to determine whether a signal component of the receive signal comprising the periodic pattern may indicate a locally stationary motion. An MTI may be chosen and adapted such that the periodic pattern and its evolving nature is detected and that the corresponding signal component is extracted.

The apparatus 100 may thus enable a discrimination between a locally stationary motion such as a breathing motion of a static person from deterministic, stationary or random motions such as a slowly moving or vibrating object. For instance, both static people and slowly moving objects may exhibit a very similar micro-Doppler signature and may therefore conventionally be complicated to distinguish.

The apparatus 100 may make use of statistic characteristics of the receive signal distinguishing such similar micro-Doppler signatures: A signal component, e.g., micro-Doppler component, of the receive signal caused by respiratory activity may be locally stationary signal since it exhibits periodic patterns that are slowly changing over time. Inanimate slowly moving or vibrating objects may exhibit micro-Doppler patterns that are stochastic (random) in nature.

The apparatus 100 may suppress stochastic and stationary micro-Doppler signals, while preserving locally stationary ones. For instance, a micro-Doppler signal caused by a fan may be stationary signal but may be suppressed by the apparatus 100 since, unlike the respiratory pattern, it doesn't evolve over time and hence its time-domain average may have zero-amplitude (caused by destructive interference between the individual MTI channels).

The apparatus 100 may therefore enable a detection of presence of static living beings in the field of view of the sensor and may prevent false alarms caused by stationary or stochastic motions. Unlike conventional systems, the apparatus 100 may dispense with masking certain regions of the field of view where a slowly moving or vibrating object is assumed to be located which may hide any information about this region and the vicinity of such object.

In the following, examples are given for a detection of a breathing motion in the field of view of the sensor based on an MTI. The processing circuitry 120 is configured to process the data 130 through selecting at least two frames from a plurality of frames of the data 130 based on the predefined breathing rate and feeding the selected frames into the MTI. For example, a frame rate - which defines the time intervals of the plurality of frames - may have a known relation to the predefined breathing rate, e.g., such that a certain number of frames correspond to the predefined breathing rate or represent in average one breathing cycle. For instance, the frame selection may be aligned to the known relation between the frame rate and the predefined breathing rate. In this manner, the signal component representing the breathing motion may be extracted or amplified whereas static objects in the field of view of the sensor may be attenuated.

Further details about the frame selection are given in the following: The processing circuitry 120 may, in some examples, be configured to select the at least two frames to have a predefined number of consecutive frames of the plurality of frames in between. Alternatively or optionally, the processing circuitry 120 may be configured to select the at least two frames by selecting every n-th frame of the plurality of frames based on the predefined breathing rate (with $n \geq 2$). For instance, the predefined number of consecutive frames in between or n may be chosen according to the known relation between the frame rate and the predefined breathing rate. The MTI may thus be a slow-rate MTI which may discard at least one frame of the data 130. For instance, the processing circuitry 120 may collect only one frame every 4 seconds to be may fed into the MTI.

In some examples, the processing circuitry 120 is configured to select one of the at least two frames from a first subset of the plurality of frames which represent an inhale interval of the breathing motion and another one of the at least two frames from a second subset of the plurality of frames which represent an exhale interval of breathing motion based on the predefined breathing rate, e.g., such that one of the selected frames lies within an inhale interval of the breathing motion and another one within an exhale interval or such that the time between two of the selected frames is half the time of one breathing cycle of the breathing motion (or a multiple thereof). This may enable the apparatus 100 to make use of the phase shift between the inhale and exhale interval. For example, if the frame rate corresponds to the predefined breathing rate such that a first frame lies within a half of the breathing cycle (e.g., an inhale interval of the breathing motion) and a second frame lies within the other half of the breathing cycle (e.g., an exhale interval), the first and the second frame may be selected by the processing circuitry 120 from the plurality of frames. In another example where the frame rate is 5 frames per second and the predefined breathing rate is 1 breathing cycle per second, the processing circuitry 120 may select every 10-th frame to align with the predefined breathing rate.

The MTI may, for example, be configured to superimpose the selected frames constructively, e.g., by negating the value of the selected frames which correspond to one of the inhale or the exhale interval, while maintaining the original sign of the value of the selected frames which correspond to the other one of the inhale or the exhale interval. This may enable an amplification of the signal component representing the breathing motion by taking into account the 180 degrees phase shift between exhale and inhale interval.

For example, the MTI may be a double delay line canceller (3-pulse canceller), and the processing circuitry 120 may be configured to select at least three frames from the plurality of frames based on the predefined breathing rate, e.g., such that two frames lie within one of the inhale interval and the exhale interval and one frame lies within the other one of the inhale and the exhale interval. Then, the MTI may process the selected frames such that an output y(t) of the MTI at time instance t is defined by Equation 17:

$$y(t) = x(t) - m \cdot x(t-1) + x(t-2)$$

Equation 17, where x(t) is a first selected frame of time instance t, x(t-1) is a second selected frame of time instance t-1 (before time instance t), x(t-2) is a third selected frame of time instance t-2 (before time instance t-1), and m is an amplification factor, e.g., 2.

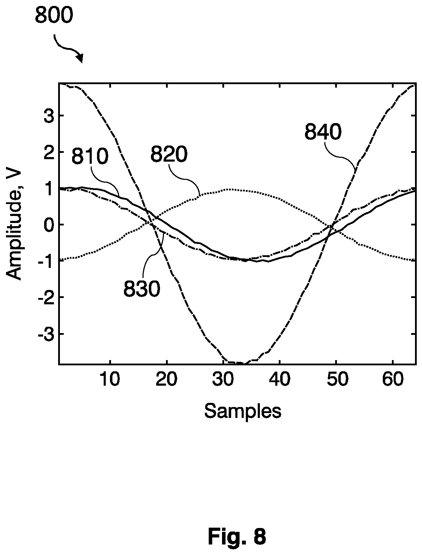

For instance, the first frame and the third frame are selected to be in phase and the second frame is selected to be 180 degrees out of phase relative to the first and third frame but is shifted to phase by negating. The coherent addition of the values of the three frames may ideally lead to ~(m+2)-times signal amplification and significant SNR (signal-to-noise ratio) improvement (e.g., up to +30 dB (decibel) and, yet static objects may be attenuated or cancelled since they do not exhibit a phase difference. The latter is illustrated by **Fig. 8** which shows an example of an amplitude curve 800 of samples of a first frame 810, a second frame 820 and a third frame 830 which are fed into an example of an MTI which thereupon provides an output 840. The frames 810, 820, 830 represent a respective sine wave of a signal component of a receive signal of a sensor. The first frame 810 and the third frame 830 are selected to be in-phase while being out-of-phase with the second frame 820. The MTI provides the output 840 based on Equation 17 with an amplification factor of 2. Thus, the output 840 may be a 4-times amplified signal with respect to one of the frames 810, 820, 830.

An example of a selection of frames for the example shown in Fig. 8 is illustrated by **Fig. 9.** Fig. 9 illustrates an example of a plurality of consecutive frames 900 of data indicating a receive signal of a sensor. The plurality of frames 900 are illustrated by blocks lined up into a row. Each of the blocks symbolizes a respective frame of the plurality of frames 900. A subset 910 of the plurality of frames 900 comprises all consecutive frames, here 20 frames, lying within a time interval of 4 seconds. The first frame 810 may be selected as the first block 811 of the subset 910, the second frame 820 may be selected as the 11-th block 912 of the subset 910 and the third frame 830 may be selected as the 21-st block 913 of the subset 910. In the example of Fig. 9, an MTI is used to process the data. The MTI comprises a summing node 920 into which the selected blocks (frames) 911, 912, 913 are fed and which may provide an output, e.g., based on Equation 17. The MTI may be further configured to discard the frames between the first, second and third frame 810, 820, 830.

In some examples, the MTI may be a multi-channel MTI comprising a plurality of channels. The processing circuitry 120 may be configured to feed the selected frames into a first channel of the plurality of channels of the MTI, like explained above. The processing circuitry 120 may be further configured to process the data 130 through selecting at least two further frames from the plurality of frames of the data 130 based on the predefined breathing rate and feeding the selected further frames into a second channel of the plurality of channels. For instance, the processing circuitry 120 may select the further frames in a similar manner like the other selected frames, e.g., with a predefined number of frames between the further frames or by selecting every n-th frame or alike. The MTI may further process the further frames in a similar manner, e.g., based on a delay line canceller such as by Equation 17, and provide an output for each channel of the plurality of channels. The processing circuitry 120 may further be configured to select one of the at least two further frames from consecutive frames in between the at least two frames. The multi-channel approach may increase the reliability of the breathing motion detection.

If the frame rate is not (completely) aligned with the predefined breathing rate (e.g., when the actual breathing rate deviates too much from the predefined breathing rate or the selected frames happen to represent a turning point of the breathing motion at the time of change from inhale to exhale interval), the micro-Doppler signal component of the breathing motion may happen to be cancelled by the slow-rate MTI and the target may be potentially lost. Therefore, a more distributed selection of frames processed in different channels may enable the apparatus 100 to prevent an undesired cancellation of the locally stationary signal and to increase the detection accuracy. Even if a single channel may be asynchronous with the predefined breathing rate, remaining channels may still be able to pick up the phase differences which brings extra reliability and better utilization of the data 130.

Further, the multi-channel MTI may, in some examples, process all frames of the plurality frames, i.e., without discarding a frame. This may further increase the reliability of detection of the apparatus 100. For example, the processing circuitry 120 may be configured to process the data 130 (e.g., indicating a time-domain representation of a (e.g., radar) receive signal) through feeding each of the plurality of frames to one of a plurality of channels of the MTI. The latter is illustrated by **Fig. 10** which shows an example of a plurality of consecutive frames 1000 of data indicating a receive signal of a sensor. The plurality of frames 1000 are illustrated by blocks lined up into a row. Each of the blocks symbolizes a respective frame of the plurality of frames 1000. In the example of Fig. 10, a time delay between adjacent channels may be 0.2 seconds, the frame rate may be 5 frames per seconds.

A first frame 1011-1 may be selected as the first block of the row, a second frame 1012-1 may be selected as the 11-th block of the row and a third frame 1013-1 may be selected as the 21-st block of the row. Thus, the first frame 1011-1 and the second frame 1012-1 as well as the second frame 1012-1 and the third frame 1013-1 have each 8 frames in between. In other examples, the number of consecutive frames in between may differ from the one illustrated by Fig. 10. The number of in-between frames may be $k \geq 1$ and may depend on, e.g., the frame rate of the data.

In the example of Fig. 10, an MTI is used to process the data. The MTI comprises a first channel 1020-1 into which the selected blocks (frames) 1011-1, 1012-1 and 1013-1 are fed. The first channel 1020-1 provides a first output, e.g., based on Equation 17, based on the input frames 1011-1, 1012-1 and 1013-1.

A fourth frame 1011-2 may be selected as the second block of the row, a fifth frame 1012-2 may be selected as the 11-th block of the row and a seventh frame 1013-2 may be selected as the 22-nd block of the row. The fourth frame and the fifth frame may therefore be selected from the in-between frames of the first and second frame 1011-1, 1012-1. The MTI comprises a second channel 1020-2 into which the selected frames 1011-2, 1012-2 and 1013-2 are fed. The second channel 1020-2 provides a second output based on the input frames 1011-2, 1012-2 and 1013-2.

The MTI of Fig. 10 has in total 10 channels comprising the first channel and the second channel 1020-1, 1020-2. A similar procedure is followed for the remaining 8 channels: Each channel is fed with respective frames such that each of the plurality of frames 1000 is fed into one of the 10 channels of the MTI. Each channel provides a respective output. The channels may therefore be fed with frames of a sliding window with a predefined frame selection pattern. The sliding window may make a pre-selection of frames for a certain channel of the plurality of channels of the MTI, for instance, a pre-selection of the first 21 frames, and continue with a pre-selection for the next channel, for instance, a pre-selection of frames from the second to the 22-nd frame, and so on. In each channel, the frames may be separated by 2 seconds. Thus, for three frames, 4 seconds may be needed to completely update the said channel. This multi-channel approach may, instead of discarding intermediate frames, use intermediate frames to form additional slow-rate MTI channels (e.g., y1, y2, etc.).

In other examples than the one shown in Fig. 10, some frames may be disregarded, only two frames or more than three frames may be fed into a channel, the frame rate may be more or less than 5 frames per second or the MTI may have less or more than 10 channels.

The MTI of Fig. 10 further comprises a summing node 1030 to which all the outputs of the individual channels are input to provide a combined output. Such a combined output may, e.g., be an average over the plurality of channels. The direct averaging over the channels in a time-domain may improve the attenuation of stochastic micro-Doppler signal components. However, in other examples, the channel outputs may firstly be transformed into a spectral representation (frequency-domain), e.g., a range representation of the receive signal, and combined afterwards. The latter may be beneficial if no random signal suppression is required. The averaging over the channels is explained further in the following.

Referring back to Fig. 1, the processing circuitry 120 is, in some examples, further configured to process the receive signal through averaging over the plurality of channels of the MTI. There may be two different approaches to average over the plurality of channels, thus, the processing circuitry 120 may be configured to average over the plurality of channels of the moving target indicator by applying averaging on a time-domain representation or a frequency-domain representation of an output of the plurality of channels: For example, the processing circuitry 120 may directly average over the MTI channels in a time-domain of the channel outputs. The processing circuitry 120 may then process the averaged output for range determination. This approach may be beneficial for mitigating an effect of a curtain or a vibrating object in the receive signal. In other examples, the processing circuitry 120 may process each MTI channel individually for extracting a range (independently) for each channel based on a respective channel output. The processing circuitry 120 may then average over the plurality of channels in a frequency-domain of the channel outputs.

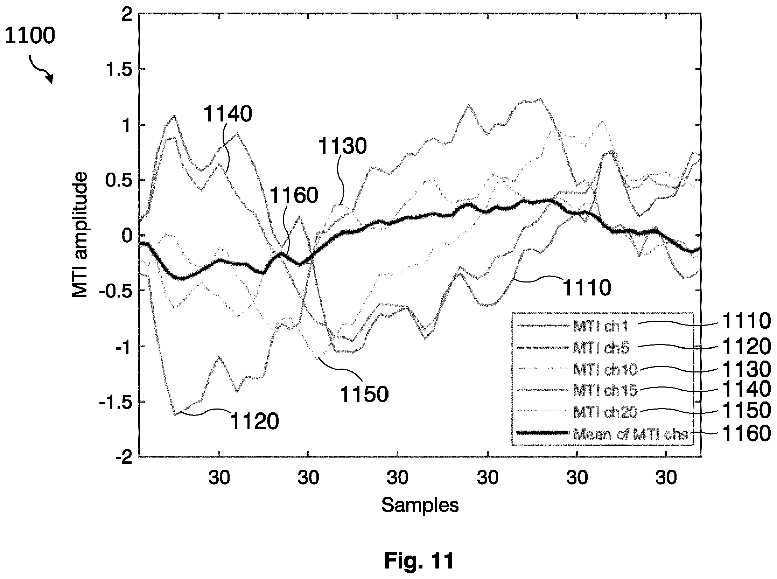

An example of averaging on a time-domain representation of an output of channels of an MTI is illustrated by **Fig. 11** which shows an example of an output 1100 of 5 MTI channels 1110, 1120, 1130, 1140 and 1150 as well as a mean 1160 of the output 1100. The 5 MTI channels 1110, 1120, 1130, 1140 and 1150 may have been fed with respective frames of data indicating a receive signal of a sensor. The receive signal may comprise signal components of a locally stationary motion in a field of view of the sensor. The mean 1160 may be determined over a long time-series, e.g., over 20 channels (including the 5 channels 1110, 1120, 1130, 1140 and 1150) or over 8 seconds. The mean 1160 shows that the locally stationary signal components of the receive signal - which are amplified by the MTI - exhibit oscillatory sine-wave patterns which reveals the locally stationary nature of the signal components. For instance, the oscillatory nature of the mean 1160 may indicate a breathing motion which has causes a phase shift in the receive signal evolving over time.

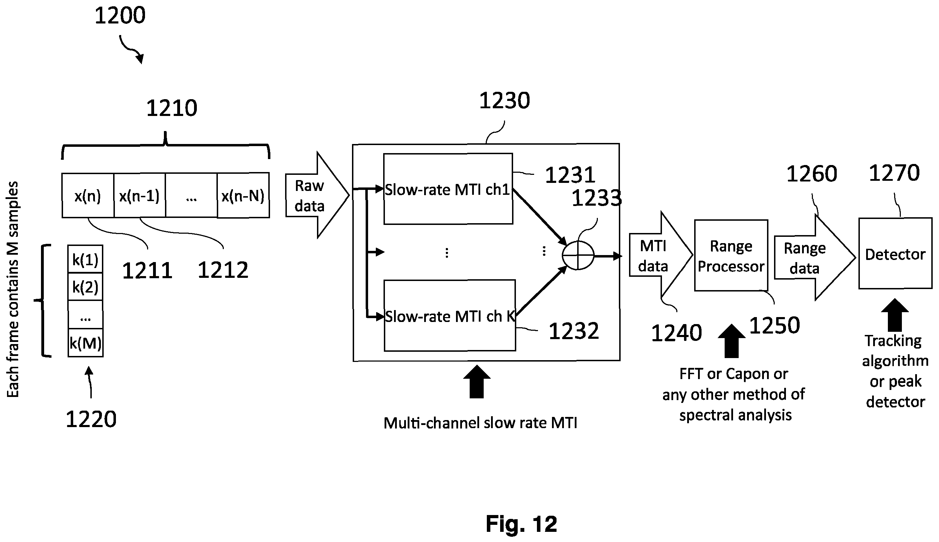

The apparatus 100 may be useful for several target applications. For example, the processing circuitry 120 may be further configured to detect a motion in a field of view of the sensor causing the locally stationary micro-Doppler signal component based on the processed receive signal. Additionally or alternatively, the apparatus 100 may be used for random micro-Doppler suppression (in case of a stochastic process), for deterministic micro-Doppler suppression (e.g., attenuating a signal component caused by a motion of a fan in the field of view of the sensor), or additional macro-Doppler processing (e.g., detection of moving persons or objects). Alternatively, the apparatus 100 may provide the processed data 130 to an external device which performs the above detection. Another example of an apparatus 1200 comprising a detector is illustrated by **Fig. 12.**

The apparatus 1200 comprises processing circuitry. The apparatus 1200 may optionally further comprise interface circuitry configured to receive data 1210 indicating a receive signal of a sensor. Alternatively, the processing circuitry may determine (at least partially) the data 1210. The data 1210 comprises a plurality of frames, e.g., frames 1211 and 1212. Each frame of the plurality of frames comprises a plurality of samples, e.g., the frame 1211 comprises samples 1220. The data 1210 may be considered raw data of the sensor.

The apparatus 1200 further comprises processing circuitry configured to process the data 1210 through isolating a locally stationary signal component of the receive signal from at least one of a stochastic and a deterministic signal component of the receive signal. The processing circuitry processes the data 1210 through selecting at least two frames from the plurality of frames of the data 1210 based on a predefined breathing rate of a breathing motion in a field of view of the sensor, and feeding the selected frames into an MTI 1230 (a multi-channel slow-rate MTI).

The MTI 1230 comprises a plurality of channels, e.g., a first channel 1231 and a second channel 1232. The processing circuitry feeds the selected frames into the first channel 1231. The processing circuitry further processes the data 1210 through selecting at least two further frames from the plurality of frames of the data 1210 and feeding the selected further frames into the second channel 1232.

The MTI 1230 further comprises a summing node 1233 which averages over the plurality of channels of the MTI 1230 by applying averaging on a time-domain representation of an output of the plurality of channels. The summing node 1233 outputs MTI data 1240 which is a combined output of the plurality of channels.

The processing circuitry feeds the MTI data 1240 into a range processor 1250 which transforms the MTI data 1240 into range data 1260, e.g., based on a fast Fourier transformation, a Capon method or alike. The processing circuitry then feeds the range data 1260 into the detector 1270. The detector 1270 may use a tracking algorithm or peak detection to detect a motion causing the locally stationary signal component.

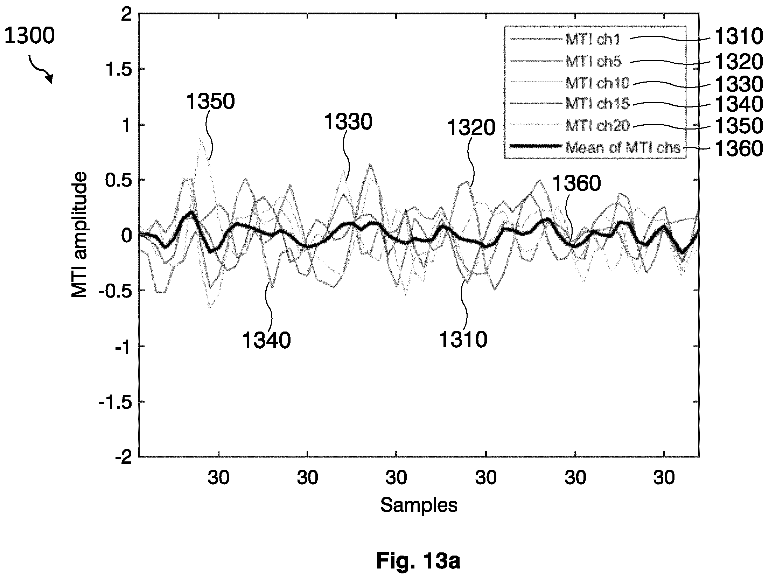

Referring back to Fig. 1, the apparatus 100 may be used in several scenarios. For instance, in a first scenario a slowly moving object (e.g., a curtain) may be present in the field of view of the sensor. The object may exhibit a random micro-Doppler pattern. An example of an output 1300 of a multi-channel MTI as described herein is illustrated by **Figs. 13a to 13c.** Figs. 13a to 13c illustrate the output 1300 for 5 channels 1310, 1320, 1330, 1340 and 1350 of the MTI as well as a combined output 1360 of a plurality of channels of the MTI. The output 1300 is based on frames of data indicating a receive signal of a sensor.

In Fig. 13a, the slowly moving (inanimate) object is the only moving object in the field of view of the sensor. The output 1300 in Fig. 13a therefore shows that a signal component of the receive signal caused by the slow movement of the object has a random instantaneous phase. The average (combined output) 1360 in time-domain thus substantially results in a zero-amplitude signal. The slowly moving object will hence not appear on a range map.

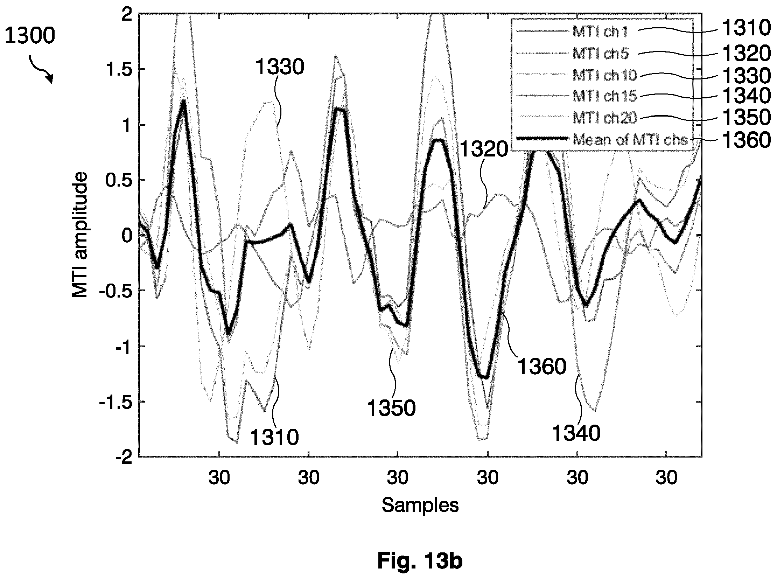

In Fig. 13b, a second object is present which is moving within the field of view of the sensor causing a macro-Doppler signal component in the receive signal. The combined output 1360 exhibits several peaks (cycles of the sine-wave) indicating the motion of the second object.

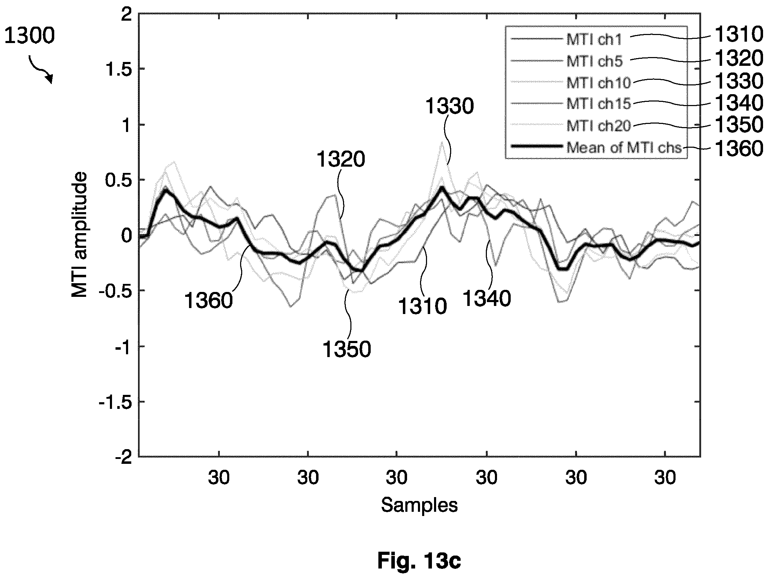

In Fig. 13c, a person is present in the field of view of the sensor who is breathing and thereby causing a micro-Doppler signal component in the receive signal. The combined output 1360 exhibits a periodic pattern which indicates the respiratory pattern. The breathing motion of the person is picked up by a detector and, thus, a stationary target (human) may be monitored.

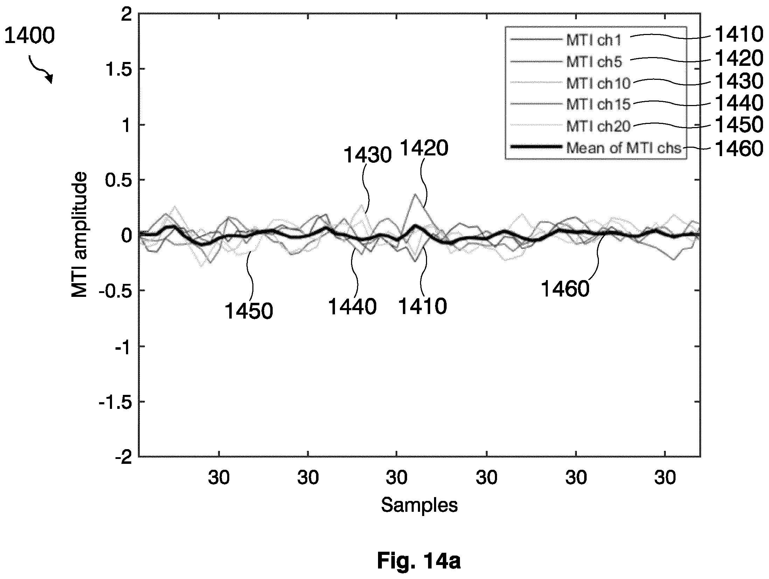

In a second scenario, a fan is present in the field of view of the sensor which causes a micro-Doppler signal component in the receive signal. An example of an output 1400 of a multi-channel MTI as described herein is illustrated by **Figs. 14a to 14c.** Figs. 14a to 14c illustrate the output 1400 for 5 channels 1410, 1420, 1430, 1440 and 1450 of the MTI as well as a combined output 1460 of a plurality of channels of the MTI. The output 1400 is based on frames of data indicating the receive signal.

In Fig. 14a, the fan is off and the field of view of the sensor is static. The combined output 1460 therefore is substantially zero.

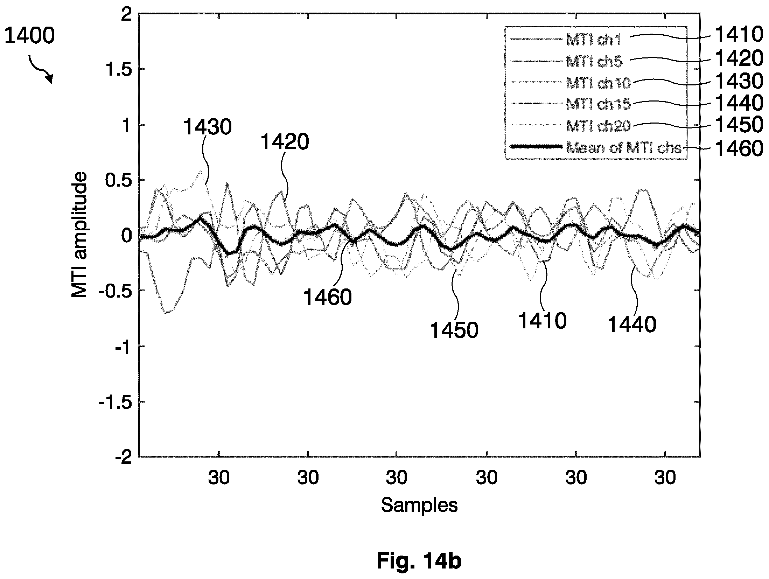

In Fig. 14b, the fan is on. The channels 1410, 1420, 1430, 1440 and 1450 show a clear signal and, thus, detect the fan motion, whereas the combined output 1460 averages out the micro-Doppler signal component of the fan motion, yielding a zero-amplitude. The attenuation of the micro-Doppler signal component may be due to the deterministic nature of the fan motion, i.e., its phase does not evolve, hence, it may be averaged out by a multi-channel slow-rate MTI.

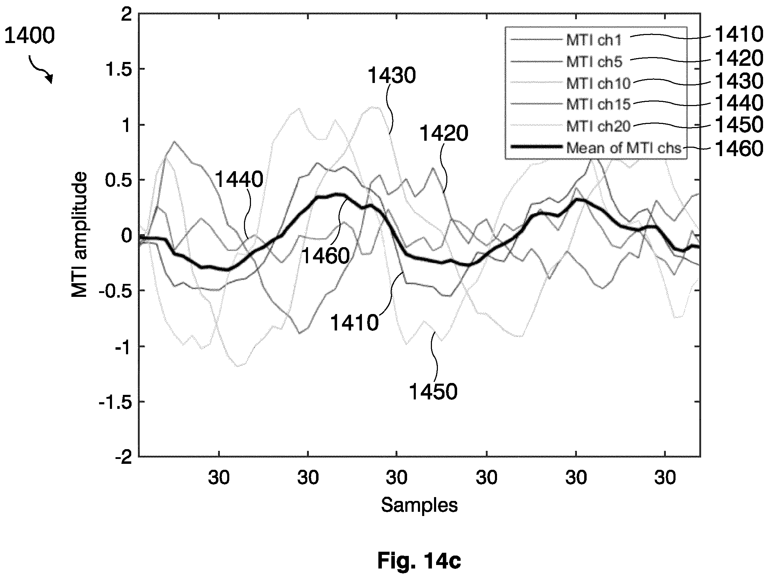

In Fig. 14c, a person is additionally present in the field of view of the sensor. The combined output 1460 exhibits a periodic pattern indicating a respiratory activity of the person.

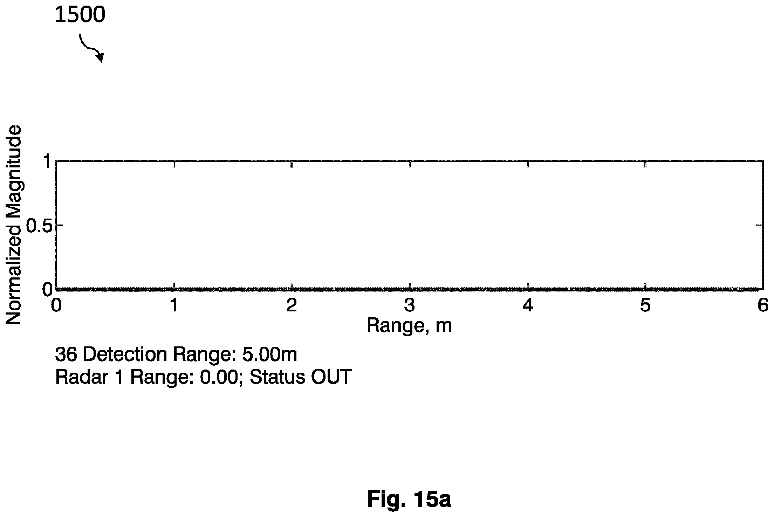

In a third scenario, a fan and a waving curtain are present in the field of view of the sensor. Each of the movement cause a micro-Doppler signal component in a receive signal of a sensor. An example of a range representation of a combined output 1500 of a multi-channel MTI as described herein is illustrated by **Figs. 15a to 15d.**

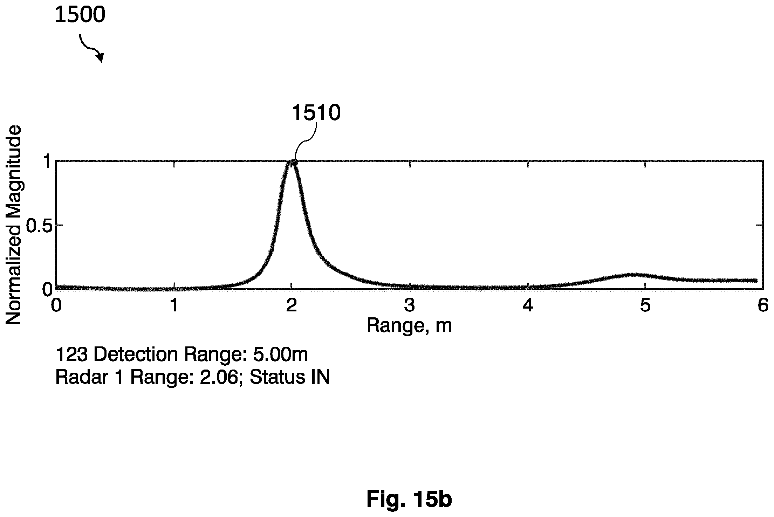

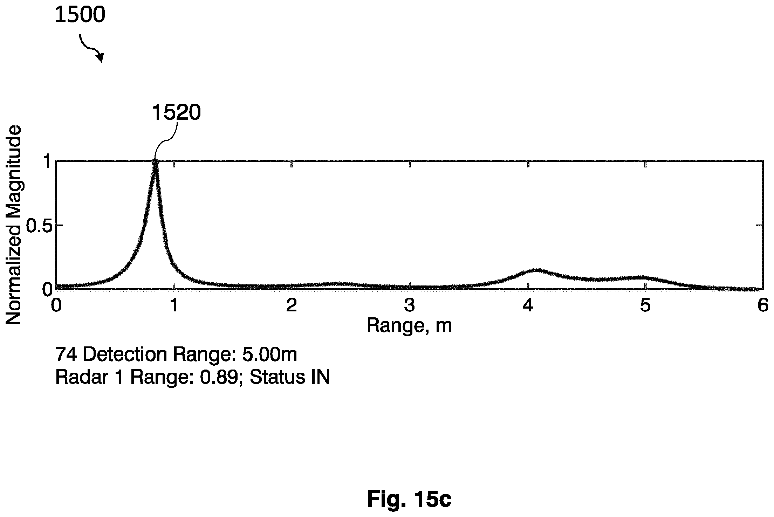

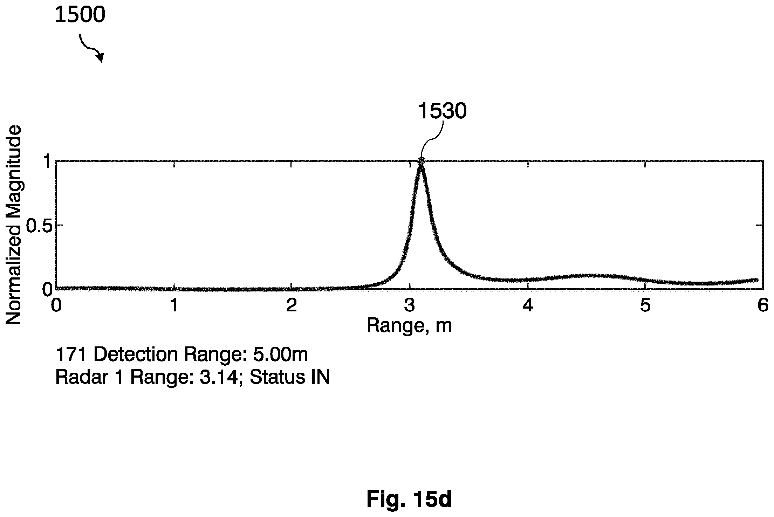

In Fig. 15a, the fan is on, and the curtain is waving, thus, the combined output 1500 is zero. In Fig. 15b, a static person is additionally present in the field of view of the sensor in a range of 2 meters. A breathing motion of the person is detected, causing a peak 1510 in the combined output 1500 at a range of 2 meters. In Fig. 15c, a static person is additionally present in the field of view of the sensor in a range of 0,8 meters. A breathing motion of the person is detected, causing a peak 1520 in the combined output 1500 at a range of 0,8 meters. In Fig. 15d, a static person is additionally present in the field of view of the sensor in a range of 3,2 meters. A breathing motion of the person is detected, causing a peak 1530 in the combined output 1500 at a range of 3,2 meters.

Apparatuses as described herein may provide a reliable discrimination of static people from slowly moving or vibrating object in a receive signal of a sensor based on the nature of their micro-Doppler signals. The apparatuses may detect a micro-Doppler signal due to respiratory activity due to its locally stationary signal component in the receive signal. The apparatuses may attenuate slowly moving or vibrating objects with a micro-Doppler pattern that are stochastic in nature (random). Further, the apparatuses may suppress stochastic and stationary micro-Doppler signals, while preserving locally stationary ones.

**Fig. 16** illustrates an example of a sensor system 1600 comprising an apparatus 1610 as described herein, such as apparatus 100, and the sensor 1620. The sensor 1620 is configured to transmit a transmit signal into a field of view of the sensor 1620 and generate the receive signal based on received reflections of the transmitted transmit signal. For example, the sensor 1620 may be at least one of a radar sensor, a lidar sensor, an optical time-of-flight sensor, a sonar sensor and an ultrasonic sensor.

Although the apparatus 1610 and the sensor 1620 are depicted as separate blocks in Fig. 16, in other examples, the apparatus 1610 may in part or in entirety be included in the sensor 1620, which thus correspondingly includes all or part of the processing circuitry (e.g., processing circuitry 120) of the apparatus 1610.

In case the apparatus 1610 is only partially included in the sensor 1620, the sensor system 1600 may include distributed processing circuitry carrying out respective parts of the processing steps, e.g., in the form of first processing (sub-) circuitry included in the sensor 1620, and second processing (sub-) circuitry external to the sensor and in communication with the first processing circuitry through interface circuitry (e.g., interface circuitry 110), for instance, for exchange of data between the first and the second processing circuitry.

In case the apparatus 1610 is integrated in the sensor 1620, the processing circuitry and the sensor 1620 may be jointly integrated in a single semiconductor chip, or in more than one semi-conductor chip.

In case the apparatus 1610 is not included in the sensor 1620, the processing circuitry may take the form of circuitry external to the sensor 1620, and may be communicatively coupled therewith through interface circuitry.

More details and aspects of the system 1600 are explained in connection with the proposed technique or one or more examples described above, e.g., with reference to Fig. 1. The system 1600 may comprise one or more additional optional features corresponding to one or more aspects of the proposed technique, or one or more examples described above.

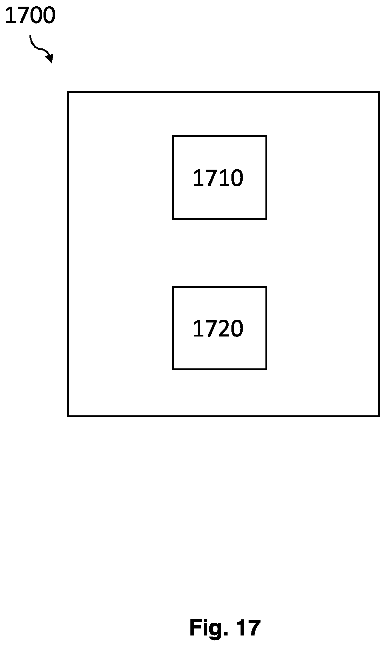

**Fig. 17.** illustrates an electronic device 1700 comprising the sensor system 1710 as described herein, such as the sensor system 1600, and control circuitry 1720 configured to control an operation of the electronic device 1700 based on the processed data.

The control circuitry 1720 may be a single dedicated processor, a single shared processor, or a plurality of individual processors, some of which or all of which may be shared, a digital signal processor (DSP) hardware, an application specific integrated circuit (ASIC) or a field programmable gate array (FPGA). The control circuitry 1720 may optionally be coupled to, e.g., read only memory (ROM) for storing software, random access memory (RAM) and/or non-volatile memory.

The electronic device 1700 may be any device with a sensing, e.g., ranging, function. The electronic device 1700 may be, e.g., a consumer device. The electronic device 1700 may be, e.g., an audio equipment such as a speaker or a telecommunication device such as a television receiver. Alternatively, the electronic device 1700 may be a medical device, e.g., for sensing vital signs of a living being. For instance, the sensor system 1710 may be configured to detect presence of a user of the electronic device 1700.

The control circuitry 1720 may control the operation of the electronic device 1700, e.g., by activating or deactivating a certain function of the electronic device 1700 based on the processed data, e.g., a certain function may be activated if it is determined that a user of the electronic device 1700 is present. For instance, the control circuitry 1720 may, if it is determined that a user is close, automatically play a video or prevent the electronic device 1700 to change into standby. Or, the control circuitry 1720 may, if it is determined that a breathing motion of the user is irregular or has stopped, output a warning signal.

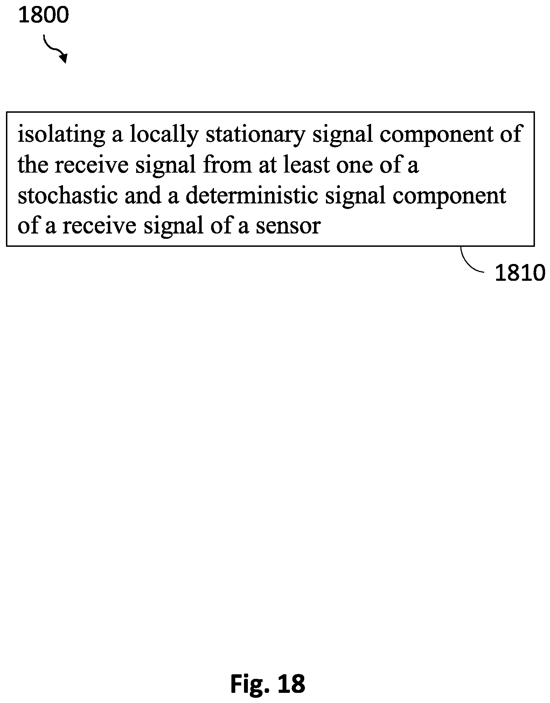

**Fig. 18** illustrates an example of a method 1800. The method 1800 comprises processing data indicating a receive signal of a sensor through isolating 1810 a locally stationary signal component of the receive signal from at least one of a stochastic and a deterministic signal component of the receive signal. The locally stationary signal component indicates a breathing motion in a field of view of the sensor. The processing of the data comprises selecting at least two frames from a plurality of frames of the data based on a predefined breathing rate assumed for the breathing motion and feeding the selected frames into a moving target indicator.

More details and aspects of the method 1800 are explained in connection with the proposed technique or one or more examples described above, e.g., with reference to Fig. 1. The method 1800 may comprise one or more additional optional features corresponding to one or more aspects of the proposed technique, or one or more examples described above.

Apparatuses and methods as described herein may provide a reliable discrimination of static people from slowly moving or vibrating object in a receive signal of a sensor based on the nature of their micro-Doppler signals. For instance, a micro-Doppler signal due to respiratory activity may be detected due to its locally stationary signal component in the receive signal. Slowly moving or vibrating objects with a micro-Doppler pattern that are stochastic in nature (random) may be attenuated. Stochastic and stationary micro-Doppler signals may be suppressed, while locally stationary ones may be preserved.

Examples may further be or relate to a (computer) program including a program code to execute one or more of the above methods when the program is executed on a computer, processor or other programmable hardware component. Thus, steps, operations or processes of different ones of the methods described above may also be executed by programmed computers, processors or other programmable hardware components. Examples may also cover program storage devices, such as digital data storage media, which are machine-, processor- or computer-readable and encode and/or contain machine-executable, processor-executable or computer-executable programs and instructions. Program storage devices may include or be digital storage devices, magnetic storage media such as magnetic disks and magnetic tapes, hard disk drives, or optically readable digital data storage media, for example. Other examples may also include computers, processors, control units, (field) programmable logic arrays ((F)PLAs), (field) programmable gate arrays ((F)PGAs), graphics processor units (GPU), application-specific integrated circuits (ASICs), integrated circuits (ICs) or system-on-a-chip (SoCs) systems programmed to execute the steps of the methods described above.

It is further understood that the disclosure of several steps, processes, operations or functions disclosed in the description or claims shall not be construed to imply that these operations are necessarily dependent on the order described, unless explicitly stated in the individual case or necessary for technical reasons. Therefore, the previous description does not limit the execution of several steps or functions to a certain order. Furthermore, in further examples, a single step, function, process or operation may include and/or be broken up into several sub-steps, - functions, -processes or -operations.

If some aspects have been described in relation to a device or system, these aspects should also be understood as a description of the corresponding method. For example, a block, device or functional aspect of the device or system may correspond to a feature, such as a method step, of the corresponding method. Accordingly, aspects described in relation to a method shall also be understood as a description of a corresponding block, a corresponding element, a property or a functional feature of a corresponding device or a corresponding system.

Claims

1. An apparatus (100), comprising processing circuitry (120) configured to process data (130) indicating a receive signal of a sensor through isolating a locally stationary signal component of the receive signal from at least one of a stochastic and a deterministic signal component of the receive signal,

   wherein the locally stationary signal component indicates a breathing motion in a field of view of the sensor, and **characterized in that** the processing circuitry (120) is configured to process the data (130) through:

   selecting at least two frames from a plurality of frames of the data (130) based on a predefined breathing rate assumed for the breathing motion; and
   feeding the selected frames into a moving target indicator.

2. The apparatus (100) of claim 1, wherein the processing circuitry (120) is configured to isolate the locally stationary signal component from the at least one of the stochastic and the deterministic signal component by extracting the locally stationary signal component of the receive signal.

3. The apparatus (100) of any one of the previous claims, wherein the processing circuitry (120) is configured to isolate the locally stationary signal component from the at least one of the stochastic and the deterministic signal component by at least one of attenuating, filtering, suppressing and removing the at least one of the stochastic and the deterministic signal component of the receive signal.

4. The apparatus (100) of any one of the previous claims, wherein the processing circuitry (120) is configured to process the data (130) through using at least one of a linear predictor, a trained machine-learning model and a moving target indicator.

5. The apparatus (100) of any one of the previous claims, wherein the at least one of the stochastic and the deterministic signal component indicates at least one of a random motion and a vibration in a field of view of the sensor.

6. The apparatus (100) of any one of the previous claims, wherein the processing circuitry (120) is configured to select one of the at least two frames from a first subset of the plurality of frames which represent an inhale interval of the breathing motion and another one of the at least two frames from a second subset of the plurality of frames which represent an exhale interval of the breathing motion based on the predefined breathing rate.

7. The apparatus (100) of any one of the previous claims, wherein the processing circuitry (120) is configured to select at least three frames from the plurality of frames based on the predefined breathing rate, and wherein the moving target indicator is a double delay line canceller.

8. The apparatus (100) of any one of the previous claims, wherein the processing circuitry (120) is configured to feed the selected frames into a first channel of a plurality of channels of the moving target indicator, and wherein the processing circuitry (120) is further configured to process the data (130) through:

   selecting at least two further frames from the plurality of frames of the data (130) based on the predefined breathing rate; and
   feeding the selected further frames into a second channel of the plurality of channels.

9. The apparatus (100) of claim 8, wherein the processing circuitry (120) is configured to select one of the at least two further frames from frames in between the at least two frames.

10. A sensor system (1600), comprising:

    an apparatus (1610) according to any one of claims 1 to 9; and
    the sensor (1620), wherein the sensor (1620) is configured to:

    transmit a transmit signal into a field of view of the sensor (1620); and
    generate the receive signal based on received reflections of the transmitted transmit signal.

11. An electronic device (1700), comprising:

the sensor system (1710) according to claim 10; and
control circuitry (1720) configured to control an operation of the electronic device based on the processed data.

12. A computer-implemented method (1800), comprising:

processing data indicating a receive signal of a sensor through isolating a locally stationary signal component of the receive signal from at least one of a stochastic and a deterministic signal component of the receive signal, wherein the locally stationary signal component indicates a breathing motion in a field of view of the sensor, and **characterized in that** processing the data comprises:

selecting at least two frames from a plurality of frames of the data based on a predefined breathing rate assumed for the breathing motion; and
feeding the selected frames into a moving target indicator.

13. The method (1800) of claim 12, further comprising isolating the locally stationary signal component from the at least one of the stochastic and the deterministic signal component by extracting the locally stationary signal component of the receive signal.

14. A non-transitory machine-readable medium having stored thereon a program having a program code for performing the method according to claim 12 or 13, when the program is executed on a processor, which is configured according to the processing circuitry (120) as set out in claim 1.

15. A program having a program code for performing the method according to claim 12 or 13, when the program is executed on a processor, which is configured according to the processing circuitry (120) as set out in claim 1.

**Patentansprüche**

1. Einrichtung (100), die eine Verarbeitungsschaltung (120) umfasst, die konfiguriert ist zum Verarbeiten von Daten (130), die ein Empfangssignal eines Sensors durch ein Isolieren einer lokal stationären Signalkomponente des Empfangssignals von mindestens einer von einer stochastischen und einer deterministischen Signalkomponente des Empfangssignals anzeigen,

wobei die lokal stationäre Signalkomponente eine Atmungsbewegung in einem Blickfeld des Sensors anzeigt, und
**dadurch gekennzeichnet, dass** die Verarbeitungsschaltung (120) konfiguriert ist zum Verarbeiten der Daten (130) durch:

Auswählen von mindestens zwei Frames aus einer Mehrzahl von Frames der Daten (130) basierend auf einer vordefinierten Atmungsrate, die für die Atmungsbewegung angenommen wird; und
Einspeisen der ausgewählten Frames in einen Indikator von beweglichen Zielen.

2. Einrichtung (100) nach Anspruch 1, wobei die Verarbeitungsschaltung (120) konfiguriert ist zum Isolieren der lokal stationären Signalkomponente von der mindestens einen der stochastischen und der deterministischen Signalkomponente, indem die lokal stationäre Signalkomponente des Empfangssignals extrahiert wird.

3. Einrichtung (100) nach einem der vorhergehenden Ansprüche, wobei die Verarbeitungsschaltung (120) konfiguriert ist zum Isolieren der lokal stationären Signalkomponente von der mindestens einen der stochastischen und der deterministischen Signalkomponente, durch mindestens eines von einem Abschwächen, Filtern, Unterdrücken und Entfernen der mindestens einen der stochastischen und der deterministischen Signalkomponente des Empfangssignals.

4. Einrichtung (100) nach einem der vorhergehenden Ansprüche, wobei die Verarbeitungsschaltung (120) konfiguriert ist zum Verarbeiten der Daten (130) durch ein Verwenden von mindestens einem von einem linearen Prädiktor, einem trainierten Maschinenlernmodell und einem Indikator von beweglichen Zielen.

5. Einrichtung (100) nach einem der vorhergehenden Ansprüche, wobei die mindestens eine der stochastischen und der deterministischen Signalkomponente mindestens eine von einer Zufallsbewegung und einer Vibration in dem Blickfeld des Sensors anzeigt.

6. Einrichtung (100) nach einem der vorhergehenden Ansprüche, wobei die Verarbeitungsschaltung (120) konfiguriert ist zum Auswählen, basierend auf der vordefinierten Atmungsrate, eines der mindestens zwei Frames aus einer ersten Teilgruppe der Mehrzahl von Frames, die ein Einatmungsintervall der Atmungsbewegung repräsentieren, und eines anderen der mindestens zwei Frames aus einer zweiten Teilgruppe der Mehrzahl von Frames, die ein Ausatmungsintervall der Atmungsbewegung repräsentieren.

7. Einrichtung (100) nach einem der vorhergehenden Ansprüche, wobei die Verarbeitungsschaltung (120) konfiguriert ist zum Auswählen, basierend auf der vordefinierten Atmungsrate, von mindestens drei Frames aus der Mehrzahl von Frames und wobei der Indikator von beweglichen Zielen ein doppelter Verzögerungsleitungsunterdrücker ist.

8. Einrichtung (100) nach einem der vorhergehenden Ansprüche, wobei die Verarbeitungsschaltung (120) konfiguriert ist zum Einspeisen der ausgewählten Frames in einen ersten Kanal aus einer Mehrzahl von Kanälen des Indikators von beweglichen Zielen, und wobei die Verarbeitungsschaltung (120) ferner konfiguriert ist zum Verarbeiten der Daten (130) durch:

   Auswählen von mindestens zwei weiteren Frames aus der Mehrzahl von Frames der Daten (130) basierend auf einer vordefinierten Atmungsrate; und
   Einspeisen der ausgewählten weiteren Frames in einen zweiten Kanal aus der Mehrzahl von Kanälen.

9. Einrichtung (100) nach Anspruch 8, wobei die Verarbeitungsschaltung (120) konfiguriert ist zum Auswählen von einem der mindestens zwei weiteren Frames zwischen den mindestens zwei Frames.

10. Sensorsystem (1600) umfassend:

    eine Einrichtung (1610) nach einem der Ansprüche 1 bis 9; und
    den Sensor (1620), wobei der Sensor (1620) konfiguriert ist zum:

       Senden eines Sendesignals in ein Blickfeld des Sensors (1620); und
       Erzeugen des Empfangssignals basierend auf empfangenen Reflexionen des gesendeten Sendesignals.

11. Elektronische Vorrichtung (1700), umfassend:

    das Sensorsystem (1710) nach Anspruch 10; und
    eine Steuerschaltung (1720), die konfiguriert ist zum Steuern eines Betriebs der elektronischen Vorrichtung basierend auf den verarbeiteten Daten.

12. Computerimplementiertes Verfahren (1800), umfassend:

    Verarbeiten von Daten, die ein Empfangssignal eines Sensors durch ein Isolieren einer lokal stationären Signalkomponente des Empfangssignals von mindestens einer von einer stochastischen und einer deterministischen Signalkomponente des Empfangssignals anzeigen,
    wobei die lokal stationäre Signalkomponente eine Atmungsbewegung in einem Blickfeld des Sensors anzeigt, und
    **dadurch gekennzeichnet, dass** das Verarbeiten der Daten umfasst:

       Auswählen von mindestens zwei Frames aus einer Mehrzahl von Frames der Daten basierend auf einer vordefinierten Atmungsrate, die für die Atmungsbewegung angenommen wird; und
       Einspeisen der ausgewählten Frames in einen Indikator von beweglichen Zielen.

13. Verfahren (1800) nach Anspruch 12, das ferner ein Isolieren der lokal stationären Signalkomponente von der mindestens einen der stochastischen und der deterministischen Signalkomponente umfasst, indem die lokal stationäre Signalkomponente des Empfangssignals extrahiert wird.

14. Nicht-flüchtiges maschinenlesbares Medium, in dem ein Programm gespeichert ist, das einen Programmcode zum Durchführen des Verfahrens nach Anspruch 12 oder 13 aufweist, wenn das Programm von einem Prozessor ausgeführt wird, der gemäß der Verarbeitungsschaltung (120) nach Anspruch 1 konfiguriert ist.

**15.** Programm, das einen Programmcode zum Durchführen des Verfahrens nach Anspruch 12 oder 13 aufweist, wenn das Programm von einem Prozessor ausgeführt wird, der gemäß der Verarbeitungsschaltung (120) nach Anspruch 1 konfiguriert ist.

**Revendications**

**1.** L'invention concerne un appareil (100) comprenant un circuit de traitement (120) configuré pour traiter des données (130) indiquant un signal de réception d'un capteur en isolant une composante de signal localement stationnaire du signal de réception par rapport à au moins une composante parmi une composante de signal stochastique et une composante de signal déterministe du signal de réception,

dans lequel la composante de signal localement stationnaire indique un mouvement respiratoire dans un champ de vision du capteur, et
le circuit de traitement (120) étant **caractérisé en ce qu'**il est configuré pour traiter les données (130) :

en sélectionnant au moins deux trames dans une pluralité de trames des données (130) sur la base d'une fréquence respiratoire prédéfinie supposée pour le mouvement respiratoire ; et
en introduisant les trames sélectionnées dans un indicateur de cible mobile.

**2.** Appareil (100) selon la revendication 1, dans lequel le circuit de traitement (120) est configuré pour isoler la composante de signal localement stationnaire par rapport à l'au moins une composante parmi la composante de signal stochastique et la composante de signal déterministe en extrayant la composante de signal localement stationnaire du signal reçu.

**3.** Appareil (100) selon l'une quelconque des revendications précédentes, dans lequel le circuit de traitement (120) est configuré pour isoler la composante de signal localement stationnaire par rapport à l'au moins une composante parmi la composante de signal stochastique et la composante de signal déterministe par au moins une action parmi une atténuation, un filtrage, une suppression et une élimination de l'au moins une composante parmi la composante de signal stochastique et la composante de signal déterministe du signal reçu.

**4.** Appareil (100) selon l'une quelconque des revendications précédentes, dans lequel le circuit de traitement (120) est configuré pour traiter les données (130) en utilisant au moins un élément parmi un prédicteur linéaire, un modèle d'apprentissage machine entraîné et un indicateur de cible mobile.

**5.** Appareil (100) selon l'une quelconque des revendications précédentes, dans lequel l'au moins une composante parmi la composante de signal stochastique et la composante de signal déterministe indique au moins élément parmi un mouvement aléatoire et une vibration dans un champ de vision du capteur.

**6.** Appareil (100) selon l'une quelconque des revendications précédentes, dans lequel le circuit de traitement (120) est configuré pour sélectionner l'une des au moins deux trames d'un premier sous-ensemble de la pluralité de trames qui représentent un intervalle d'inspiration du mouvement respiratoire et une autre des au moins deux trames d'un deuxième sous-ensemble de la pluralité de trames qui représentent un intervalle d'expiration du mouvement respiratoire sur la base du rythme respiratoire prédéfini.

**7.** Appareil (100) selon l'une quelconque des revendications précédentes, dans lequel le circuit de traitement (120) est configuré pour sélectionner au moins trois trames dans la pluralité de trames sur la base de la fréquence respiratoire prédéfinie, et dans lequel l'indicateur de cible mobile est un annuleur à double ligne à retard.

**8.** Appareil (100) selon l'une quelconque des revendications précédentes, dans lequel le circuit de traitement (120) est configuré pour introduire les trames sélectionnées dans un premier canal d'une pluralité de canaux de l'indicateur de cible mobile, et dans lequel le circuit de traitement (120) est en outre configuré pour traiter les données (130) :

en sélectionnant au moins deux autres trames dans la pluralité de trames de données (130) sur la base de la fréquence respiratoire prédéfinie ; et
en introduisant les trames supplémentaires sélectionnées dans un deuxième canal de la pluralité de canaux.

**9.** Appareil (100) selon la revendication 8, dans lequel le circuit de traitement (120) est configuré pour sélectionner l'une des au moins deux trames supplémentaires parmi des trames situées entre les au moins deux trames.

10. Système de capteur (1600), comprenant :

un appareil (1610) selon l'une quelconque des revendications 1 à 9 ; et
le capteur (1620), le capteur (1620) étant configuré pour :

transmettre un signal de transmission dans un champ de vision du capteur (1620) ; et
générer le signal de réception en fonction des réflexions reçues du signal de transmission transmis.

11. Dispositif électronique (1700), comprenant :

le système de capteur (1710) selon la revendication 10 ; et
un circuit de commande (1720) configuré pour commander un fonctionnement du dispositif électronique sur la base des données traitées.

12. Procédé mis en œuvre par ordinateur (1800), comprenant l'étape consistant à :

traiter des données indiquant un signal de réception d'un capteur en isolant une composante de signal localement stationnaire du signal de réception par rapport à au moins une composante parmi une composante de signal stochastique et une composante de signal déterministe du signal de réception,
dans lequel la composante de signal localement stationnaire indique un mouvement respiratoire dans un champ de vision du capteur, et
le traitement des données est **caractérisé en ce qu'**il consiste à :

sélectionner au moins deux trames dans une pluralité de trames des données sur la base d'une fréquence respiratoire prédéfinie supposée pour le mouvement respiratoire ; et
en introduisant les trames sélectionnées dans un indicateur de cible mobile.

13. Procédé (1800) selon la revendication 12, comprenant en outre l'isolation de la composante de signal localement stationnaire par rapport à l'au moins une composante parmi la composante de signal stochastique et la composante de signal déterministe en extrayant la composante de signal localement stationnaire du signal reçu.

14. Support non transitoire lisible par machine sur lequel est stocké un programme comportant un code de programme permettant de réaliser le procédé selon la revendication 12 ou 13 lorsque le programme est exécuté sur un processeur, le programme étant configuré conformément au circuit de traitement (120) décrit dans la revendication 1.

15. Programme comportant un code de programme pour exécuter le procédé selon la revendication 12 ou 13, lorsque le programme est exécuté sur un processeur, le programme étant configuré conformément au circuit de traitement (120) décrit dans la revendication 1.

100

110

130

120

**Fig. 1**

**Fig. 2a**

**Fig. 2b**

**Fig. 3a**

EP 4 443 191 B1

Fig. 3b

**Fig. 3c**

EP 4 443 191 B1

Fig. 3d

Fig. 4a

**Fig. 4b**

EP 4 443 191 B1

Fig. 5a

Fig. 5b

**Fig. 6a**

**Fig. 6b**

700

**Fig. 7a**

700

**Fig. 7b**

Fig. 8

**Fig. 9**

EP 4 443 191 B1

**Fig. 10**

Fig. 11

Fig. 12

Each frame contains M samples

Fig. 13a

**Fig. 13b**

**Fig. 13c**

EP 4 443 191 B1

Fig. 14a

Fig. 14b

**Fig. 14c**

1500

36 Detection Range: 5.00m
Radar 1 Range: 0.00; Status OUT

**Fig. 15a**

EP 4 443 191 B1

Fig. 15b

1500

1520

74 Detection Range: 5.00m
Radar 1 Range: 0.89; Status IN

**Fig. 15c**

EP 4 443 191 B1

171 Detection Range: 5.00m
Radar 1 Range: 3.14; Status IN

**Fig. 15d**

EP 4 443 191 B1

1600

1610

1620

**Fig. 16**

1700

**Fig. 17**

1800

isolating a locally stationary signal component of
the receive signal from at least one of a
stochastic and a deterministic signal component
of a receive signal of a sensor

1810

**Fig. 18**

**REFERENCES CITED IN THE DESCRIPTION**

**Patent documents cited in the description**

- WO 2022101390 A2
- US 2015369911 A1
- CN 115015867 A
- CN 109581343 A